# EUROPEAN PATENT APPLICATION

(11) **EP 2 778 227 A1**
(43) Date of publication of application: **17.09.2014**
(21) Application number: 12847315.4
(22) Date of filing: 09.11.2012
(51) Int. Cl.: C12P 7/46, C12P 7/62

(54) **METHOD FOR PRODUCING SUCCINIC ACID**

(30) Priority: 11.11.2011 JP 2011248101
(71) Applicant: Mitsubishi Chemical Corporation, Chiyoda-ku Tokyo 100-8251 (JP)
(72) Inventor: YUNOMURA, Shuichi, Aoba-ku, Yokohama-shi, Kanagawa 227-8502 (JP); KIDO, Daisuke, Aoba-ku, Yokohama-shi, Kanagawa 227-8502 (JP); AOYAMA, Ryusuke, Aoba-ku, Yokohama-shi, Kanagawa 227-8502 (JP); MURASE, Makoto, Aoba-ku, Yokohama-shi, Kanagawa 227-8502 (JP)
(74) Representative: Vossius & Partner
(86) International application number: PCT/JP2012/079172
(87) International publication number: WO 2013/069786

(57) **Abstract**

A method for producing succinic acid comprising:
(A) a step of converting an organic raw material to succinic acid in the presence of a microorganism or a processed product thereof in an aqueous medium and
(B) a step of recovering the succinic acid, wherein (A) the step of converting an organic raw material to succinic acid comprises:
(a1) a step of adjusting the concentration of a succinic acid alkali metal salt in the aqueous medium to 80 mM or more; and then,
(a2) a step of adding ammonia and/or an ammonium salt in the aqueous medium.

## Description

### TECHNICAL FIELD

The present invention relates to a method for producing succinic acid using a microorganism.

### BACKGROUND ART

Aliphatic dicarboxylic acids such as succinic acid and adipic acid are widely used as raw materials for polymers such as polyester and polyamide and as synthetic raw materials for foods, pharmaceuticals, and other chemical products. Currently these aliphatic dicarboxylic acids are produced industrially from raw materials derived from fossil fuel resources. Meanwhile, techniques for deriving aliphatic dicarboxylic acids from biomass resources are drawing attention in view of global-scale environmental problems such as recent concerns about fossil fuel resource depletion and increased carbon dioxide in the atmosphere.

When succinic acid is produced by fermentation, pH decreases along with the production of succinic acid. Thus, usually, reaction is carried out while adjusting pH by neutralization.

Well-known neutralizers used for pH adjustment in the fermentation production of succinic acid are compositions of basic compounds, such as ammonia, ammonium carbonate, urea, alkali metal or alkali earth metal hydroxides, carbonate, and bicarbonate. Neutralization using compositions of such basic compounds is known to be able to prevent growth inhibition and metabolism activity reduction in microorganisms, and death thereof due to pH decrease, thereby preventing the reduction of succinic acid productivity.

Patent Literature 1 describes a method for producing a non-amino organic acid such as succinic acid while neutralizing with magnesium carbonate and/or magnesium hydroxide in an aqueous medium containing a certain concentration range of monovalent cations.

Patent Literature 2 describes a method for producing an organic acid such as succinic acid by neutralization with a sodium compound and/or a potassium compound in a microorganism culture growth step and an organic acid production reaction step.

Non-Patent Literature 1 describes a method for producing an organic acid such as succinic acid by adding sodium bicarbonate in advance while neutralizing with ammonia water.

Non-Patent Literature 2 describes a method for producing an organic acid such as succinic acid by the changing of a neutralizer from sodium hydroxide to ammonia water in a continuous culture system with microorganism recycle.

### PRIOR ART REFERENCES

### Patent Literature

Patent Literature 1: WO 2005/026349
Patent Literature 2: JP-A-2006-197821

### Non-Patent Literature

Non-Patent Literature 1: Appl Microbiol Biotechnol. 2005 Sep; 68 (4): 475-480.
Non-Patent Literature 2: J. Microbiol. Biotechnol. 2008 Jul; 18 (7): 1252-1256.

### SUMMARY OF THE INVENTION

The fermentation production of succinic acid requires highly concentrated accumulation of succinic acid. However, there is a problem in that succinic acid production rate reduces as succinic acid accumulates to higher concentrations, because of influences of stress due to increased osmotic pressure, metabolism activity reduction due to product inhibition, and the like.

The method described in Patent Literature 1 uses magnesium carbonate and/or magnesium hydroxide as a neutralizer. However, since these compounds are poorly water-soluble, precise pH control is difficult. Accordingly, the method has a problem in terms of maintaining pH in an optimum range for allowing a microorganism to produce succinic acid.

The method described in Patent Literature 2 is a method for improving succinic acid productivity by a specific combination of neutralizers used in the microorganism culture growth step and the organic acid production reaction step. The method is, however, still problematic because of production rate reduction along with the highly concentrated accumulation of succinic acid.

The method described in Non-Patent Literature 1 is a method for improving succinic acid productivity by adding sodium bicarbonate in advance to supply bicarbonate ions suitable for succinic acid production. While the method produces succinic acid while adjusting pH with ammonia water, there still remains the problem that succinic acid production rate reduces as it accumulates to higher concentrations.

The method described in Non-Patent Literature 2 makes pH adjustment by changing a plurality of kinds of neutralizers in order to investigate an appropriate neutralizer for a continuous culture system with microorganism recycle. Although productivity is substantially stable at low succinic acid concentration, there is a problem about succinic acid productivity in the highly concentrated accumulation of succinic acid.

The neutralization methods described in Patent Literature 1 and 2 and Non-Patent Literature 1 and 2 above are all those for preventing pH reduction to improve succinic acid productivity. However, these methods have room for improvement in production rate under the condition of highly concentrated succinic acid, and, therefore, a neutralization technique for higher productivity has been desired.

It is an object of the present invention to provide a method for producing succinic acid more efficiently by preventing pH reduction in a fermentation solution to improve production rate under the condition of highly concentrated succinic acid.

The present inventors conducted extensive and intensive research to solve the above problem and consequently found that succinic acid production rate increases by specifying the concentration range of a succinic acid alkali metal salt in an aqueous medium and then adding ammonia and/or an ammonium salt in a step of converting an organic raw material to succinic acid in the presence of a microorganism or a processed product thereof in the aqueous medium, thereby completing the present invention.

Specifically, the present invention provides the following inventions:
[1] A method for producing succinic acid comprising:
   (A) a step of converting an organic raw material to succinic acid in the presence of a microorganism or a processed product thereof in an aqueous medium and
   (B) a step of recovering the succinic acid, wherein (A) the step of converting an organic raw material to succinic acid comprises:
      (a1) a step of adjusting the concentration of a succinic acid alkali metal salt in the aqueous medium to 80 mM or more; and then,
      (a2) a step of adding ammonia and/or an ammonium salt in the aqueous medium.
[2] The method for producing succinic acid according to the [1], wherein (a1) the step of adjusting the concentration of a succinic acid alkali metal salt in the aqueous medium to 80 mM or more is a step of neutralizing the aqueous medium with an alkali metal salt and/or an alkali metal hydroxide.
[3] The method for producing succinic acid according to the [1] or [2], wherein in (a1) the step of adjusting the concentration of a succinic acid alkali metal salt in the aqueous medium to 80 mM or more, the concentration of the succinic acid alkali metal salt is 1300 mM or less.
[4] The method for producing succinic acid according to any of the [1] to [3], wherein the succinic acid alkali metal salt is disodium succinate and/or dipotassium succinate.
[5] The method for producing succinic acid according to any of the [1] to [4], wherein the microorganism is at least one selected from the group consisting of coryneform bacteria, *Escherichia coli*, the genus *Anaerobiospirillum*, the genus *Actinobacillus,* filamentous fungi, and yeast.
[6] The method for producing succinic acid according to any of the [1] to [5], wherein the microorganism is a microorganism modified such that lactate dehydrogenase activity is reduced compared to an unmodified strain.
[7] The method for producing succinic acid according to any of the [1] to [6], wherein the microorganism is a microorganism modified such that pyruvate carboxylase activity is enhanced compared to an unmodified strain.
[8] The method for producing succinic acid according to any of the [1] to [7], wherein the aqueous medium contains at least one selected from the group consisting of a carbonate ion, a bicarbonate ion, and carbon dioxide gas.
[9] The method for producing succinic acid according to any of the [1] to [8], wherein (A) the step of converting an organic raw material to succinic acid is performed in an anaerobic atmosphere.
[10] The method for producing succinic acid according to any of the [1] to [9], wherein (A) the step of converting an organic raw material to succinic acid is performed by batch reaction or semi-batch reaction.
[11] The method for producing succinic acid according to any of the [1] to [10], wherein the organic raw material is glucose and/or sucrose.
[12] A method for producing a succinic acid-containing polymer comprising a step of producing succinic acid by the method according to any of the [1] to [11] and a step of polymerizing the obtained succinic acid.

In the production of succinic acid by fermentation process, the present invention can increase production rate under the condition of highly concentrated succinic acid, resulting in reduction in the cost of succinic acid production. In addition, the invention can greatly contribute to the solving of environmental problems, problems of fossil fuel resource depletion, and the like.

### MODES FOR CARRYING OUT THE INVENTION

Embodiments of the present invention will be described in detail hereinbelow.

### <Microorganism Used in the Present Invention>

The microorganism used in the present invention is not particularly limited as long as it is a microorganism having an ability to produce succinic acid.

In the present invention, the phrase "an ability to produce succinic acid" means that when the microorganism is cultured in a culture medium, it can produce and accumulate succinic acid in the culture medium.

The microorganism used in the present invention may be an organism inherently having the ability to produce succinic acid or an organism provided with the ability to produce succinic acid by breeding.

Examples of means for providing the ability to produce succinic acid by breeding include mutation processing and genetic recombination, and a known method can be employed, such as enhanced expression of an enzyme gene in a biosynthetic pathway of succinic acid. Examples of the known method for providing the ability to produce succinic acid include modification for reducing lactate dehydrogenase activity described later and means for enhancing pyruvate carboxylase activity.

In addition, the present invention can use a processed product of bacterial cells of a microorganism. Examples of the processed product of a microorganism include immobilized bacterial cells obtained by immobilizing the bacterial cells of a microorganism with acrylamide, carrageenan, or the like, crushed bacterial cells, a centrifugal supernatant thereof, and a fraction obtained by partial purifying the supernatant by ammonium sulfate treatment or the like.

The microorganism used in the present invention is not particularly limited as long as it has the ability to produce succinic acid, and examples of the microorganism include a microorganism selected from the group consisting of coryneform bacteria, *Escherichia coli*, the genus *Anaerobiospirillum*, the genus *Actinobacillus,* filamentous fungi, and yeast. Among these, preferred are coryneform bacteria, *Escherichia coli,* the genus *Anaerobiospirillum*, the genus *Actinobacillus,* and yeast, more preferred are coryneform bacteria, *Escherichia coli*, and yeast, and particularly preferred are coryneform bacteria.

A coryneform bacterium used in the present invention is not particularly limited as long as it is classified as a coryneform bacterium. Examples of the coryneform bacterium include a bacterium belonging to the genus *Corynebacterium*, a bacterium belonging to the genus *Brevibacterium*, and a bacterium belonging to the genus *Arthrobacter*. Among these, preferred is a bacterium belonging to the genus *Corynebacterium* or *Brevibacterium,* and more preferred is a bacterium classified as *Corynebacterium glutamicum, Brevibacterium flavum, Brevibacterium ammoniagenes*, or *Brevibacterium lactofermentum*.

Particularly preferred specific examples of the coryneform bacterium used in the present invention include *Brevibacterium flavum* MJ-233 (FERM BP-1497), MJ-233 AB-41 (FERM BP-1498), *Brevibacterium ammoniagenes* ATCC6872, *Corynebacterium glutamicum* ATCC31831, and *Brevibacterium lactofermentum* ATCC13869. At present, *Brevibacterium flavum* may be classified into *Corynebacterium glutamicum* (Lielbl W, Ehrmann M, Ludwig W, Schleifer KH, Int J Syst Bacteriol., 1991, Vol. 41, p255-260). In the present invention, *Brevibacterium flavum* MJ-233 strain and a mutant strain thereof MJ-233 AB-41 strain, respectively, are regarded as the same strains as *Corynebacterium glutamicum* MJ-233 strain and MJ-233 AB-41 strain, respectively.

*Brevibacterium flavum* MJ-233 has been deposited as of April 28, 1975, with National Institute of Bioscience and Human Technology, Agency of Industrial Science and Technology, Ministry of International Trade and Industry (currently, International Patent Organism Depositary, National Institute of Advanced Industrial Science and Technology; Tsukuba Central 6, 1-1-1 Higashi, Tsukuba, Ibaraki 305-8566, Japan) under the accession No. FERM P-3068, and converted to an international deposit under the accession No. FERM BP-1497 as of May 1, 1981, under the Budapest Treaty.

In addition, the above microorganism may be any strain among those such as not only a wild type strain but a mutant strain obtained by an ordinary mutation processing such as UV irradiation or NTG treatment, and a recombinant strain derived by a genetic method such as cell fusion or gene recombination, and the like.

Examples of a bacterium belonging to the genus *Anaerobiospirillum* to be used in the present invention include *Anaerobiospirillum succiniciproducens.*

Examples of a bacterium belonging to the genus *Actinobacillus* to be used in the present invention include *Actinobacillus succinogenes.*

Examples of a filamentous fungus to be used in the present invention include those belonging to the genus *Aspergillus*, *Penicillium*, and *Rhizopus*. Among them, examples of bacteria belonging to the genus *Aspergillus* include *Aspergillus niger* and *Aspergillus oryzae*, and examples of bacteria belonging to the genus *Penicillium* include *Penicillium chrysogenum* and *Penicillium simplicissimum*. In addition, examples of bacteria belonging to the genus *Rhizopus* include *Rhizopus oryzae*.

Examples of a yeast to be used in the present invention include the genus *Saccharomyces*, *Shizosaccharomyces*, *Candida*, *Pichia*, *Kluyveromyces*, and *Zygosaccharomyces.*

Examples of the genus Saccharomyces to be used in the present invention include *Saccharomyces cerevisiae, Saccharomyces uvarum,* and *Saccharomyces bayanus*.

Examples of the genus *Shizosaccharomyces* to be used in the present invention include *Schizosaccharomyces pombe*.

Examples of the genus *Candida* to be used in the present invention include *Candida albicans*, *Candida sonorensis*, and *Candida glabrata*.

Examples of the genus *Pichia* to be used in the present invention include *Pichia pastoris* and *Pichia stipitis*.

Examples of the genus *Kluyveromyces* to be used in the present invention include *Kluyveromyces lactis*, *Kluyveromyces marxianus*, and *Kluyveromyces thermotolerans*.

Examples of the genus *Zygosaccharomyces* to be used in the present invention include *Zygosaccharomyces bailii* and *Zygosaccharomyces rouxii*.

In the production method of the present invention, it is preferable to use a microorganism modified such that lactate dehydrogenase (hereinafter referred to also as LDH) activity is reduced. The term "LDH activity" used herein means an activity catalyzing a reaction that reduces pyruvic acid to lactic acid (LDH: EC 1. 1. 1. 27). The phrase "LDH activity is reduced" means that LDH activity is reduced compared to an unmodified strain. The LDH activity is reduced to preferably 30% or less and more preferably 10% or less per unit bacterial cell weight compared to the unmodified strain. Alternatively, the LDH activity may be completely lost. The reduction of LDH activity can be confirmed by the measurement of LDH activity using a known method such as the method of Kanarek et al. (Kanarek L, Hill RL, J Biol Chem., 1964, Vol. 239, p 4202-4206). A strain having reduced LDH activity can be produced by disrupting LDH gene. Specific examples of a method for disrupting LDH gene include a method by homologous recombination into chromosome (see JP-A-H11-206385) and a method using sacB gene (Schafer A, Tauch A, Jager W, Kalinowski J, Thierbach G, Puhler A, Gene 1994 Vol. 145 (1), p69-73).

In addition, in the production method of the present invention, the microorganism may be an organism modified such that pyruvate carboxylase (hereinafter referred to also as PC) activity is enhanced. The term "PC activity" used herein means an activity catalyzing a reaction that carboxylates pyruvic acid to produce oxaloacetic acid (PC: EC 6. 4. 1. 1). The phrase "PC activity is enhanced" means that PC activity is increased compared to an unmodified strain. The PC activity is increased to preferably 1.5 times or more, and more preferably 3 times or more per unit bacterial cell weight compared to the unmodified strain. The enhancement of PC activity can be confirmed by the measurement of PC activity using a known method such as the method of Fisher et al., (Fisher SH, Magasanik B, J Bacteriol., 1984, Vol. 158 (1), p55-62).

A specific example of a method for creating a strain having enhanced PC activity is a method that can construct the strain by inducing the high expression of a pc gene using a plasmid in a host microorganism in the same manner as described in JP-A-H11-196888. Alternatively, pc gene may be incorporated in a chromosome by homologous recombination or pc gene expression may be enhanced by promoter substitution (JP-A-2008-259451). Transformation can be performed, for example, by an electric pulse method (Vertes AA, Inui M, Kobayashi M, Kurusu Y, Yukawa H, Res. Microbiol., 1993, Vol. 144 (3), p181-185) or the like.

Specific examples of the pc gene include a *Corynebacterium glutamicum-*derived pc gene (Peters-Wendisch PG, Kreutzer C, Kalinowski J, Paterk M, Sahm H, Eikmanns BJ, Microbiology, 1998, Vol. 144, p915-927). In addition, another suitably usable pc gene may be a DNA that hybridizes with the *Corynebacterium glutamicum*-derived pc gene under stringent conditions or a DNA that has a homology of 80% or more, preferably 90% or more, more preferably 95% or more, and particularly preferably 99% or more with the nucleotide sequence of the gene and encodes a protein having PC activity.

Furthermore, in the production method of the present invention, the microorganism may be a microorganism modified such that the activity of at least one enzyme selected from the group consisting of acetate kinase (hereinafter referred to also as ACK) and phosphotransacetylase (hereinafter referred to also as PTA) is reduced.

The term "PTA activity" means an activity catalyzing a reaction that transfers phosphate to acetyl-CoA to produce acetyl phosphate (PTA: EC 2. 3. 1. 8). The phrase "modified such that PTA activity is reduced" means that PTA activity is reduced compared to an unmodified strain. The PTA activity is reduced to preferably 30% or less and more preferably 10% or less per unit bacterial cell weight compared to the unmodified strain. Alternatively, the PTA activity may be completely lost. The reduction of PTA activity can be confirmed by the measurement of PTA activity using a known method such as the method of Klotzsch et al. (Klotzsch HR, Meth Enzymol., 1969, Vol. 12, p381-386).

The term "ACK activity" means an activity catalyzing a reaction that produces acetic acid from acetyl phosphate and ADP (ACK: EC 2. 7. 2. 1). The phrase "modified such that ACK activity is reduced" means that PTA activity is reduced compared to an unmodified strain. The ACK activity is reduced to preferably 30% or less and more preferably 10% or less per unit bacterial cell weight compared to the unmodified strain. Alternatively, the ACK activity may be completely lost. The reduction of ACK activity can be confirmed by the measurement of ACK activity using a known method such as the method of Ramponi et al. (Ramponi G, Meth Enzymol., 1975, Vol. 42, p409-426).

Either one of the PTA activity and the ACK activity may be reduced, but more preferably, both of the activities are reduced in order to efficiently reduce the by-production of acetic acid.

In *Corynebacterium glutamicum* (including those classified as *Brevibacterium flavum*), as described in Microbiology. 1999 Feb; 145 (Pt2): 503-513, both of the enzymes PTA and ACK are encoded by pta-ack operon (GenBank Accession No. X89084), and thus the activities of both enzymes can be reduced by disrupting pta gene.

A strain having reduced PTA and ACK activities can be obtained by disrupting the genes of PTA and ACK according to a known method such as a method using homologous recombination or a method using sacB gene (Schafer A, Tauch A, Jager W, Kalinowski J, Thierbach G, Puhler A, Gene 1994 Vol. 145 (1), p69-73). Specifically, these genes can be disrupted according to a method disclosed in JP-A-2006-000091. Examples of pta and ack genes usable include a gene having the nucleotide sequence of the GenBank Accession No. X89084 and a gene having a homology enough to cause homologous recombination with pta and ack genes on a host chromosome. The "homology enough to cause homologous recombination" used herein is preferably 80% or more, more preferably 90% or more, and particularly preferably 95% or more. In addition, homologous recombination can occur between DNA molecules hybridizable with the above genes under stringent conditions.

In addition, in the production method of the present invention, the microorganism may be a microorganism modified such that fumarate reductase (hereinafter referred to also as FRD) activity is enhanced. The term "FRD activity" used herein means an activity that catalyzes a reaction reducing fumaric acid to succinic acid (EC: 1.3.5.1, 1.3.5.4). The phrase "FRD activity is enhanced" means that FRD activity is increased compared to an unmodified strain. The FRD activity is increased to preferably 1.5 times or more and more preferably 3 times or more per unit bacterial cell weight compared to the unmodified strain. The enhancement of FRD activity can be confirmed by the measurement of FRD activity using a known method such as the method of Ackrell et al. (Ackrell BA, Kearney EB, Singer TP, Methods Enzymol., 1978, Vol. 53, p466-83).

FRD activity can be enhanced, for example, by modifying a microorganism as a parent strain through a gene recombination using frd gene, or the like. In addition, there are also cases in which a gene encoding succinate dehydrogenase (hereinafter referred to also as SDH) encodes a protein having both SDH activity and FRD activity. Accordingly, the expression of a gene encoding a protein having both FRD and SDH activities may be increased. The term "SDH activity" used herein means an activity catalyzing a reaction that oxidizes succinic acid to fumaric acid (EC 1.3.5.1, 1.3.99.1). SDH activity can be measured by a known method such as the method of Ackrell et al. (Ackrell BA, Kearney EB, Singer TP, Methods Enzymol., 1978, Vol. 53, p466-83).

A specific example of a method for creating a strain having enhanced FRD activity is a method that can construct the strain by inducing the high expression of frd gene or sdh gene using a plasmid in a host microorganism in the same manner as described in WO 2005/021770. Alternatively, frd or sdh gene may be incorporated in a chromosome by homologous recombination or the expression of frd or sdh gene may be enhanced by promoter substitution.

Specific examples of frd gene include an *Escherichia coli*-derived frd gene (WO 2005/021770). The gene is an operon gene including respective genes encoding four subunits (frdA, frdB, frdC, and frdD) forming fumarate reductase. The full length of the gene may be introduced in a microorganism as a parent strain or each subunit gene may be individually introduced. As the each subunit gene, there can be suitably used a DNA that hybridizes with the *Escherichia coli*-derived frdA, frdB, frdC, or frdD gene under stringent conditions or a DNA having a homology of 80% or more, preferably 90% or more, more preferably 95% or more, and particularly preferably 99% or more with the nucleotide sequence of the gene, as long as the DNA encodes a subunit protein that can form a complex having FRD activity.

Specific examples of a gene encoding a protein having both SDH and FRD activities include a *Corynebacterium glutamicum*-derived sdh gene (WO 2005/021770). The gene is an operon gene including respective genes that encode three subunits (sdhA, sdhB, and sdhC) forming succinate dehydrogenase. The full length of the gene may be introduced in a microorganism as a parent strain or each subunit gene may be individually introduced. As the each subunit gene, there can be suitably used a DNA that hybridizes with the *Corynebacterium glutamicum-*derived sdhA, sdhB, or sdhC gene under stringent conditions or a DNA having a homology of 80% or more, preferably 90% or more, more preferably 95% or more, and particularly preferably 99% or more with the nucleotide sequence of the gene, as long as the DNA encodes a subunit protein that can form a complex having FRD activity.

Furthermore, in the production method of the present invention, the microorganism may be a microorganism modified such that malic acid dehydrogenase (hereinafter referred to also as MDH) activity is enhanced. The term "MDH activity" used herein means an activity catalyzing a reaction that reduces oxaloacetic acid to malic acid (MDH: EC 1.1.1.37). The phrase "MDH activity is enhanced" means that MDH activity is increased compared to an unmodified strain. The MDH activity is increased to preferably 1.5 times or more and more preferably 3 times or more per unit bacterial cell weight compared to the unmodified strain. The enhancement of MDH activity can be confirmed by the measurement of MDH activity using a known method such as the method of Molenaar et al. (Molenaar D, van der Rest ME, Drysch A, Yucel R J Bacteriol., 2000, Vol. 182 (24), p6884-91).

A specific example of a method for creating a strain having enhanced MDH activity is a method that can construct the strain by inducing the high expression of mdh gene using a plasmid in a host microorganism in the same manner as described in JP-A-2006-320208. Alternatively, mdh gene may be incorporated in a chromosome by homologous recombination or the expression of mdh gene may be enhanced by promoter substitution.

Specific examples of an mdh gene usable include a *Corynebacterium glutamicum*-derived mdh gene (JP-A-2006-320208). In addition, as the mdh gene, there can be suitably used a DNA that hybridizes with the *Corynebacterium glutamicum*-derived mdh gene under stringent conditions or a DNA having a homology of 80% or more, preferably 90% or more, more preferably 95% or more, and particularly preferably 99% or more with the nucleotide sequence of the gene and encoding a protein having MDH activity.

The microorganism used in the present invention may be a bacterium obtained by combining two or more kinds of modifications from among the above-described modifications. When a plurality of modifications is performed, the order of the modifications to be performed is not limited.

### <Method for Producing Succinic Acid>

A method for producing succinic acid according to the present invention is a method for producing succinic acid comprising (A) a step of converting an organic raw material to succinic acid in the presence of a microorganism or a processed product thereof in an aqueous medium (hereinafter referred to as "step (A)") and (B) a step of recovering the succinic acid (hereinafter referred to as "step (B)", wherein the step (A) comprises:
(a1) a step of adjusting the concentration of a succinic acid alkali metal salt in the aqueous medium to 80 mM or more; and then,
(a2) a step of adding ammonia and/or an ammonium salt in the aqueous medium.

In the use of the above microorganism in the method for producing succinic acid according to the present invention, the microorganism may be slant-cultured in a solid medium such as an agar medium and then directly used in the step (A). Alternatively, before performing the step (A), the microorganism may be cultured in a liquid medium in advance if necessary. Specifically, a microorganism that has been grown in advance may be allowed to produce succinic acid. The microorganism may be grown in advance by performing seed culture and/or main culture described below.

Seed culture is performed to prepare the bacterial cells of the microorganism that are to be subjected to main culture. A medium used for seed culture can be an ordinary medium used for culture of microorganisms. For example, there can be used an ordinary medium prepared by adding natural nutrient sources such as meat extract, yeast extract, and special peptone to a composition containing inorganic salts such as ammonium sulfate, potassium phosphate, and magnesium sulfate. In addition, an organic raw material such as glucose may be added as a carbon source to the culture medium if necessary.

Additionally, seed culture is preferably performed at an ordinary optimum growth temperature. The ordinary optimum growth temperature means a temperature at which growth rate is the fastest under conditions used for succinic acid production. Specific culture temperature is usually from 25 to 40°C, and preferably from 30 to 37°C. In the case of a coryneform bacterium, culture temperature is usually from 25 to 35°C, more preferably from 28 to 33°C, and particularly preferably approximately 30°C.

In addition, seed culture is preferably performed at an ordinary optimum growth pH. The ordinary optimum growth pH means a pH at which growth rate is the fastest under conditions used for succinic acid production. Specific culture pH is usually from 4 to 10, and preferably from 6 to 8. In the case of a coryneform bacterium, culture pH is usually from 6 to 9, and preferably from 6.5 to 8.5.

In addition, culture time for seed culture is not particularly limited as long as it is a time that allows a predetermined amount of bacterial cells to be obtained. The culture time therefor is usually from 6 to 96 hours. Additionally, in seed culture, preferably, oxygen is supplied by aeration or stirring.

Bacterial cells after seed culture can be used in main culture described below. However, seed culture can be omitted, and bacterial cells slant-cultured on a solid medium such as an agar medium may be used directly in main culture. Additionally, seed culture may be repeated a plurality of times if needed.

Main culture is performed to prepare the bacterial cells of the microorganism that is to be subjected to a succinic acid production reaction described later, and the main purpose of main culture is to increase the amount of the bacterial cells. When the seed culture described above is performed, bacterial cells obtained by the seed culture are used to perform main culture.

A culture medium used for main culture can be an ordinary culture medium used for culture of microorganisms. For example, the culture medium used therefor can be an ordinary culture medium prepared by adding natural nutrient sources such as meat extract, yeast extract, and peptone to a composition containing inorganic salts such as ammonium sulfate, potassium phosphate, and magnesium sulfate.

In addition, in main culture, preferably, an organic raw material as a carbon source is added to the culture medium. The organic raw material used in main culture is not particularly limited as long as the material can be assimilated by the microorganism to be grown. Examples of organic raw materials usually used include fermentative sugars, such as carbohydrates such as galactose, lactose, glucose, fructose, sucrose, saccharose, starch, and cellulose and polyalcohols such as glycerol, mannitol, xylitol, and ribitol. Among these, glucose, sucrose, or fructose is preferable, and particularly glucose or sucrose is preferable.

In addition, a saccharified starch solution containing any of the fermentative sugars, molasses, or the like may be used. Specifically, preferably, the fermentative sugar is a sugar solution squeezed out of a plant such as sugar cane, sugar beet, or sugar maple.

These organic raw materials may be used alone or in combination thereof.

The concentration of the organic raw material to be used is not particularly limited. It is advantageous to add the organic raw material in a range that does not inhibit the growth of bacterial cells. The organic raw material can be used usually in a range of from 0.1 to 10% (W/V), and preferably in a range of from 0.5 to 5% (W/V) with respect to a culture solution. Additionally, the organic raw material may be additionally added in accordance with the reduction of the organic raw material due to the growth of bacterial cells.

In addition, main culture is preferably performed at an ordinary optimum growth temperature. Specific culture temperature is usually from 25 to 40°C, and preferably from 30 to 37°C. In the case of a coryneform bacterium, the culture temperature is usually from 25 to 35°C, more preferably from 28 to 33°C and particularly preferably approximately 30°C.

In addition, main culture is preferably performed at an ordinary optimum growth pH. Specific culture pH is usually from 4 to 10, and preferably 6 to 8. In the case of a coryneform bacterium, the culture pH is usually from 6 to 9 and preferably from 6.5 to 8.5.

In addition, culture time for main culture is not particularly limited as long as it is a time that allows a predetermined amount of bacterial cells to be obtained. The culture time therefor is usually from 6 to 96 hours. Additionally, in main culture, preferably, oxygen is supplied by aeration or stirring.

In addition, in main culture, as a method for preparing bacterial cells more suitable for succinic acid production, there can be used a method that cultures bacterial cells in such a manner as to alternately repeat the depletion and fulfillment of a carbon source during a short time, described in JP-A-2008-259451.

Bacterial cells after main culture can be used in the succinic acid production reaction described later (the step (A)). However, the culture solution may be used directly or bacterial cells recovered by centrifugation, membrane separation or the like may be used.

Succinic acid may be produced by reacting a microorganism subjected to seed culture or main culture with an organic raw material while growing the microorganism in a culture medium containing the organic raw material or by reacting bacterial cells grown by seed culture or main culture in advance with an organic raw material in a reaction solution containing the organic raw material.

Hereinafter, a description will be given of (A) the step of converting an organic raw material to succinic acid in the presence of a microorganism or a processed product thereof in an aqueous medium.

The step (A) of the present invention is characterized by including (a1) a step of adjusting the concentration of succinic acid alkali metal salt in the aqueous medium to 80 mM or more (hereinafter referred to as "step (a1)"), and then (a2) a step of adding ammonia and/or an ammonium salt in the aqueous medium (hereinafter referred to as "step (a2)").

The term "aqueous medium" used herein means an aqueous solution used for performing succinic acid production reaction, which is preferably an aqueous solution containing a nitrogen source, an inorganic salt, and the like, as described later. Succinic acid production reaction can be performed by reacting the microorganism or a processed product thereof with an organic raw material in the aqueous solution. In the present specification, the aqueous medium means the entire solution contained in a reaction vessel.

Preferable specific examples of the succinic acid alkali metal salt include disodium succinate and dipotassium succinate.

The succinic acid alkali metal salt in the aqueous medium in the step (a1) has a concentration of 80 mM or more, preferably 120 mM or more, more preferably 160 mM or more, and particularly preferably 200 mM or more. Additionally, an upper limit value of the concentration of the succinic acid alkali metal salt in the aqueous medium is not particularly limited. However, considering the tendency that succinic acid production rate reduces as succinic acid accumulates to high concentrations even though the advantageous effect of the present invention is obtained, the upper limit value thereof is preferably 1300 mM or less, more preferably 1000 mM or less, still more preferably 800 mM or less, particularly preferably 650 mM or less, and most preferably 620 mM or less.

In order to obtain the advantageous effect of the present invention, it is necessary to allow the microorganism to produce succinic acid by adding ammonia and/or an ammonium salt in a state in which the microorganism is put under a constant load given by a stress caused due to the succinic acid alkali metal salt contained in the aqueous medium. It is preferable to adjust the concentration of the succinic acid alkali metal salt in the above range in terms of giving an appropriate level of load to the microorganism.

The concentration of the succinic acid alkali metal salt may be adjusted in the above range by adding the succinic acid alkali metal salt itself in the aqueous medium or by separately adding succinic acid and an alkali metal salt or an alkali metal hydroxide to form a succinic acid alkali metal salt. In the case of separate additions, the order of additions of the components is not limited.

Herein, the alkali metal salt or the alkali metal hydroxide is not particularly limited as long as it can adjust the concentration of the succinic acid alkali metal salt in the above concentration range. Examples of the alkali metal salt or the alkali metal hydroxide include hydroxides, carbonates, and bicarbonates of sodium or potassium. Specific examples thereof include sodium hydroxide, potassium hydroxide, sodium carbonate, potassium carbonate, sodium bicarbonate, potassium bicarbonate, and mixtures thereof.

In addition, when producing succinic acid by reacting a microorganism or a processed product thereof with an organic raw material while adding an alkali metal salt or an alkali metal hydroxide in an aqueous medium, the produced succinic acid is neutralized by the alkali metal salt or the alkali metal hydroxide to produce a succinic acid alkali metal salt. Accordingly, the concentration of the succinic acid alkali metal salt in the aqueous medium may be adjusted in the above range by adding the alkali metal salt or the alkali metal hydroxide in the aqueous medium.

In this case, when performing the step (a2) after terminating the step (a1), it is enough to change the neutralizer that is to be used for pH adjustment from the alkali metal salt or the alkali metal hydroxide to ammonia and/or an ammonium salt, which is preferable in that the succinic acid alkali metal salt necessary for obtaining the advantageous effect of the present invention can also be obtained from the organic raw material. In other words, in the present invention, preferably, the microorganism or a processed product thereof is allowed to react with the organic raw material while neutralizing the aqueous solution with the alkali metal salt or the alkali metal hydroxide, whereby a succinic acid alkali metal salt is produced, and then, at a point in time where the concentration of the succinic acid alkali metal salt becomes a value of 80 mM or more, the neutralizer is changed from the alkali metal salt or the alkali metal hydroxide to ammonia and/or an ammonium salt to continue succinic acid production.

The concentration of the succinic acid alkali metal salt in the aqueous medium can be measured by performing a quantitative analysis of succinic acid and a quantitative analysis of alkali metal ion. In this case, a lower value between a concentration value of the succinic acid and a value twice the concentration of the alkali metal ion is regarded as the concentration of the succinic acid alkali metal salt. This is because succinic acid is divalent and alkali metal is monovalent. In particular, in the step (a1), in the case in which while neutralizing the aqueous medium with the alkali metal salt or the alkali metal hydroxide, the microorganism or a processed product thereof is allowed to react with the organic raw material, whereby a succinic acid alkali metal salt is produced and the concentration of the succinic acid alkali metal salt is adjusted, an organic acid other than succinic acid and the like are also usually by-produced in the aqueous medium and also neutralized by the alkali metal salt or the alkali metal hydroxide. Accordingly, the succinic acid concentration in the aqueous medium becomes lower than the value twice the concentration of the alkali metal ion. Therefore, in this case, the succinic acid concentration in the aqueous medium can be considered the concentration of the succinic acid alkali metal salt.

Examples of the quantitative analysis method for the succinic acid include an organic acid analysis method using liquid chromatography. For example, the analysis can be made under the following conditions:

### <Organic Acid Analysis>

Column: ULTRON PS-80H 8.0 mm I.D. x 30 cm, manufactured by Shinwa Chemical Industries Ltd.
Eluent: water (perchloric acid) (60% perchloric acid aqueous solution 1.8 ml/1L-H₂O)
Temperature: 60°C

Examples of the quantitative analysis method for the alkali metal ion include a cation analysis method using an ion analyzer. For example, the analysis can be made under the following conditions:

### <Cation Analysis>

Column: PCI-311S manufactured by DKK-TOA Corporation
Eluent:2L eluent for monovalent and divalent cations (6547780K) manufactured by DKK-TOA Corporation
Temperature: 40°C

In the production method of the present invention, it is important to select an alkali metal salt as a succinate that is to be contained in the aqueous medium, and to select ammonia and/or an ammonium salt as a neutralizer used for pH adjustment in succinic acid production in the aqueous medium. In other words, it is important to perform the step (a1) and then the step (a2).

Specific examples of ammonia and/or an ammonium salt to be added in the step (a2) include ammonia (ammonium hydroxide), ammonium carbonate, ammonium bicarbonate, and mixtures thereof, among which ammonia (ammonium hydroxide), ammonium bicarbonate, and mixtures thereof are preferable.

A method for adding ammonia and/or an ammonium salt is not particularly limited as long as the aqueous medium is in a pH range described below. During the step (a2), ammonia and/or an ammonium salt may be continuously added or intermittently added, for example, once every 30 minutes to 1 hour.

Even if succinic acid ammonium or a succinic acid alkali earth metal salt is used instead of the succinic acid alkali metal salt in the step (a1), it tends to be difficult to obtain the effect of enabling production rate to increase under the condition of highly concentrated succinic acid. In addition, even if a salt or a hydroxide of an alkali metal or an alkali earth metal compound is used instead of ammonia and/or an ammonium salt in the step (a2), it tends to be difficult to obtain the effect of enabling production rate to increase under the condition of highly concentrated succinic acid, although it is possible to perform pH adjustment itself of the aqueous medium.

The reason for this seems to be that a stress imposed by the succinic acid alkali metal salt and a stress imposed by the ammonia and/or ammonium salt used as the neutralizer after that cause a change in the metabolism activity of the microorganism, thereby improving succinic acid productivity. Ammonia or an ammonium salt can be assimilated through the ammonia fixation pathways of the microorganism, such as an amino acid synthesis pathway, and activation of such metabolism pathways is advantageous in terms of obtaining a reducing power. The change in the metabolism activity caused by the state of the microorganism under the stress from the succinic acid alkali metal salt seems to improve the balance between oxidation and reduction, thereby improving succinic acid productivity.

In Non-Patent Literature 1, sodium bicarbonate is added in advance. However, succinic acid is produced while neutralizing with ammonia water in a state in which the succinic acid alkali metal salt is not sufficiently present. Thus, even when a stress by the ammonia water is given to the microorganism without a stress load caused by the succinic acid alkali metal salt, it seems to be impossible to obtain the effect of enabling production rate to increase under the condition of highly concentrated succinic acid.

In Non-Patent Literature 2, in the continuous culture system with microorganism recycle, the neutralizer is changed from sodium hydroxide to ammonia water to produce succinic acid. However, the succinic acid concentration in the aqueous medium when the neutralizer has been changed is approximately 9 g/L (the sodium succinate concentration is approximately 76 mM), which is low. Thus, the microorganism is not put under any stress load from the sodium succinate necessary to obtain the advantageous effect of the present invention. It, therefore, seems to be impossible to obtain the effect of enabling production rate to increase under the condition of highly concentrated succinic acid.

Additionally, hereinafter, a description will be given of conditions for succinic acid production reaction in the step (A). The reaction conditions described below are common between the steps (a1) and (a2) unless otherwise specified.

The organic raw material to be used in the step (A) is not particularly limited as long as the material is a carbon source that allows the microorganism to assimilate it to produce succinic acid. Examples of organic raw materials usually used include fermentative sugars, such as carbohydrates such as galactose, lactose, glucose, fructose, sucrose, starch, and cellulose and polyalcohols such as glycerol, mannitol, xylitol, and ribitol. Among these, glucose, sucrose, or fructose is preferable, and particularly glucose or sucrose is preferable.

In addition, there may be used a saccharified starch solution containing any of the fermentative sugars, molasses, or the like. Specifically, preferred is a sugar solution squeezed out of a plant such as sugar cane, sugar beet, or sugar maple.

These organic raw materials may be used alone or in combination thereof. The concentration of the organic raw material to be used is not particularly limited. It is advantageous to make the concentration thereof as high as possible in a range that does not inhibit succinic acid production. The organic raw material can be used usually 5% (W/V) or more, and preferably 10% (W/V) or more, with respect to a reaction solution, whereas usually 30% (W/V) or less, and preferably 20% (W/V) or less, with respect thereto . Additionally, the organic raw material may be additionally added in accordance with the reduction of the organic raw material due to the progress of the reaction.

The organic raw material can be contained in an aqueous medium. The aqueous medium is preferably an aqueous solution containing a nitrogen source, an inorganic salt, and the like, as described above. For example, the aqueous medium may be a culture medium for culture of a microorganism or a buffer solution such as a phosphate buffer solution. The nitrogen source used herein is not particularly limited as long as it is a nitrogen source that allows the present microorganism to assimilate it to produce succinic acid. Specific examples of the nitrogen source include various organic and inorganic nitrogen compounds such as ammonium salts, nitrates, urea, soybean hydrolyzate, casein hydrolyzate, peptone, yeast extract, meat extract, and corn steep liquor. Examples of the inorganic salt usable include metal salts such as various phosphates, sulfates, magnesium, potassium, manganese, iron, and zinc. In addition, growth promoting factors, such as vitamins such as biotin, pantothenic acid, inositol, and nicotinic acid and nucleotides and amino acids are added as needed. Furthermore, in order to suppress foaming during reaction, it is desirable to add an appropriate amount of a commercially available antifoaming agent in the aqueous medium in advance.

The aqueous medium also preferably contains, for example, a carbonate ion, a bicarbonate ion, or carbon dioxide gas (carbon dioxide), in addition to the organic raw material, the nitrogen source, the inorganic salt, and the like described above. Carbonate ion or bicarbonate ion is supplied from magnesium carbonate, sodium carbonate, sodium bicarbonate, potassium carbonate, potassium bicarbonate, or the like that can also be used as a neutralizer. Carbonate ion or bicarbonate ion can also be supplied from carbonate, bicarbonate, salts thereof, or carbon dioxide gas, as needed. Specific examples of the salts of carbonate or bicarbonate include magnesium carbonate, ammonium carbonate, sodium carbonate, potassium carbonate, ammonium bicarbonate, sodium bicarbonate, and potassium bicarbonate. The concentration of the carbonate ion or the bicarbonate ion in the aqueous medium is usually 1 mM or more, preferably 2 mM or more, and more preferably 3 mM or more, as well as usually 500 mM or less, preferably 300 mM or less, and more preferably 200 mM or less. In the case of containing carbon dioxide gas, the carbon dioxide gas is contained in an amount of usually 50 mg or more, preferably 100 mg or more, and more preferably 150 mg or more per L of the aqueous medium, whereas usually 25 g or less, preferably 15 g or less, and more preferably 10 g or less per L thereof.

In the step (A), preferably, the pH of the aqueous medium is adjusted in a range that allows the activity of the microorganism to be exhibited most effectively depending on the kind of the microorganism used. Specifically, in the case of using a coryneform bacterium, desirably, the pH of the aqueous medium is usually 5.5 or more, preferably 6 or more, more preferably 6.6 or more, and still more preferably 7.1 or more, whereas usually 10 or less, preferably 9.5 or less, and more preferably 9.0 or less. Thus, even during reaction in the step (a2), it is desirable to adjust the pH of the aqueous medium in the above range by ammonia and/or an ammonium salt as needed.

The amount of bacterial cells of the microorganism to be used in the succinic acid production reaction of the step (A) is not particularly limited, but as wet cell weight, usually 1 g/L or more, preferably 10 g/L or more, and more preferably 20 g/L or more, whereas usually 700 g/L or less, preferably 500 g/L or less, and more preferably 400 g/L or less.

Time of succinic acid production reaction is not particularly limited. As a sum of the respective reaction times of the steps (a1) and (a2), the reaction time is usually 1 hour or more, and preferably 3 hours or more, whereas usually 168 hours or less, and preferably 72 hours or less. The time distribution between the step (a1) and the step (a2) is not particularly limited. Although the time distribution depends upon other conditions such as the amount of bacterial cells used in succinic acid production reaction, for example, it is preferable to perform the step (a1) for a period of time from 3 hours to 36 hours and then the step (a2) for a period of time from 3 hours to 36 hours.

Temperature of succinic acid production reaction may be the same as the optimum growth temperature of the microorganism to be used, but it is advantageous to perform the reaction at a higher temperature than the optimum growth temperature. Succinic acid production reaction is performed at a temperature usually from 2 to 20°C higher than that, and preferably from 7 to 15°C higher than that. Specifically, in the case of a coryneform bacterium, reaction temperature is usually 35°C or more, preferably 37°C or more, and more preferably 39°C or more, whereas usually 45°C or less, preferably 43°C or less, and more preferably 41°C or less. It is not necessary to constantly maintain the temperature in the range of from 35 to 45°C during the succinic acid production reaction, but it is desirable to maintain the above temperature range for a period of time of 50% or more, and preferably 80% or more of the total reaction time.

Succinic acid production rate in the step (A) is not particularly limited but usually 2 g/L/h or more, and preferably 4 g/L/h or more. In addition, the higher the succinic acid production rate, the more preferable it is. Although the production rate depends on other conditions such as the amount of bacterial cells used in succinic acid production reaction and the concentration of accumulation of succinic acid at the calculation of the rate, an upper limit value of the production rate is, for example, 40 g/L/h or less.

The succinic acid production reaction may be performed by aeration or stirring, but is preferably performed without aeration and in an anaerobic atmosphere in which no oxygen is supplied. The condition "in an anaerobic atmosphere" mentioned herein can be obtained by a method such as performing the reaction without aeration in a sealed vessel, performing the reaction while supplying an inert gas such as nitrogen gas, or aerating with an inert gas containing carbon dioxide gas.

The production method of the present invention is not particularly limited and can be applied to all of batch reaction, semi-batch reaction, and continuous reaction. In the present invention, batch reaction or semi-batch reaction is preferable in that succinic acid production rate can be improved while succinic acid is being accumulated to high concentrations.

Through the microorganism reaction described hereinabove, succinic acid (which is present as succinate in an aqueous medium, but for convenience, the succinate obtained by the step (A) is referred to as succinic acid) can be produced and accumulated in the aqueous medium. The accumulated succinic acid can be recovered by the step (B) described below.

Hereinafter, a description will be given of (B) the step of recovering succinic acid.

Succinic acid accumulated in the aqueous medium (to be exact, a succinate such as a succinic acid alkali metal salt or a succinic acid ammonium salt) can be recovered from the aqueous medium according to a usual method. Specifically, for example, solids such as bacterial cells are removed by centrifugation, filtration, or the like, and then the resulting solution is desalinated with an ion-exchange resin or the like. The desalinated solution is, for example, purified by crystal growth (crystallization) or column chromatography, thereby recovering succinic acid.

Furthermore, a succinic acid-containing polymer can be produced by performing polymerization reaction using the succinic acid obtained in the step (B) as a raw material. Under the recent circumstances where environmentally-friendly industrial products are on the increase, much attention has been paid to polymers obtained from plant-derived raw materials. Particularly, the succinic acid produced by the present invention can be used by processing into polymers such as polyester and polyamide. Specific examples of succinic acid-containing polymers include a succinic acid polyester produced by the polymerization of a diol such as butanediol or ethylene glycol with succinic acid and a succinic acid polyamide produced by the polymerization of a diamine such as hexamethylene with succinic acid.

In addition, the succinic acid obtained by the production method of the present invention or a composition containing the succinic acid can also be used in food additives, pharmaceuticals, cosmetics, and the like.

### EXAMPLES

Hereinafter, the present invention will be described in more detail using Examples. However, the invention is not limited to the Examples.

In the present Examples, regarding the concentration of succinic acid as equal to the concentration of a succinic acid alkali metal salt, the concentration of succinic acid in an aqueous medium was measured using a liquid chromatography under the following conditions. The obtained succinic acid concentration was used as the concentration of the succinic acid alkali metal salt.

### <Organic Acid Analysis>

Column: ULTRON PS-80H 8.0 mm I.D. x 30 cm, manufactured by Shinwa Processing Co., Ltd.
Eluent: water (perchloric acid) (60% perchloric acid aqueous solution 1.8 ml/1L-H₂O)
Temperature: 60°C

### [Example 1]

### < Succinic Acid Production by Sodium Succinate Addition and Ammonia Neutralization>

### (A) Seed Culture

An amount of 1000 mL of a culture medium A (4 g of urea, 14 g of ammonium sulfate, 0.5 g of potassium phosphate 1, 0.5 g of dipotassium phosphate, 0.5 g of magnesium sulfate heptahydrate, 20 mg of ferrous sulfate heptahydrate, 20 mg of manganese sulfate hydrate, 200 µg of D-biotin, 200 µg of thiamine hydrochloride, 1 g of yeast extract, 1 g of casamino acid, and 1000 mL of distilled water) was placed in a 1-L reagent bottle and heat-sterilized at 121°C for 20 minutes. After cooling down to room temperature, 15 mL of the sterilized culture medium A was placed in a 200-mL Erlenmeyer flask, and 600 µL of pre-sterilized 50% glucose aqueous solution was added. Then, MJ233/PC-4/ΔLDH strain (a PC-enhanced and LDH-disrupted strain: WO 2009/025363) was inoculated in the medium and cultured at 30°C for 5.5 hours. In a 500-mL Erlenmeyer flask was placed 100 mL of the culture medium A, and 4 mL of pre-sterilized 50% glucose aqueous solution was added thereto. The obtained culture solution was inoculated to an O.D. (660 nm) of 0.02 and seed cultured at 30°C for 20 hours.

### (B) Main Culture

A culture medium with a total amount of 2303 mL consisting of 9.88 g of a phosphoric acid aqueous solution (85 wt%), 7.32 g of potassium chloride, 4.39 g of ammonium sulfate, 2.2 g of magnesium sulfate heptahydrate, 176 mg of manganese sulfate pentahydrate, 176 mg of ferrous sulfate heptahydrate, 44.29 g of CSL (Corn Steep Liquor), 16.4 g of 10N potassium hydroxide aqueous solution, 3.76 g of an

antifoaming agent (CE457 manufactured by NOF Corporation), and distilled water was placed in a 5-L jar fermentor and heat-sterilized at 121°C for 20 minutes. After cooling down to room temperature, 22 mL of a pre-filter sterilized vitamin solution (a D-biotin and thiamine hydrochloride (each 0.2 g/L) aqueous solution), 118 mL of pre-sterilized 720 g/L glucose aqueous solution, and 148 mL of the seed culture solution described above were added to the resulting culture medium. Considering the amount of evaporation from the weights of the solution before and after the heat-sterilization, sterilized water was added to give a total amount of 2782 mL. Then, main culture was started while maintaining the temperature of the jar fermentor at 30°C and maintaining pH at 7.2 using 28% ammonia water, at a back pressure of 0.05 MPa, an aeration of 2.4 L/min, and a stirring rate of 640 rev/min. When the dissolved oxygen concentration, after having reduced down to substantially 0, started to increase again and reached 1 ppm, approximately 7 g of pre-sterilized 720 g/L glucose was added, which again reduced the dissolved oxygen concentration down to 0. Every time the dissolved oxygen concentration increased again, the addition of the glucose solution was repeated in the above-described manner, and this operation was continued for 19 hours after starting the culture.

### (C) Succinic Acid Production Reaction

An amount of 5.2 g of a phosphoric acid aqueous solution (85 wt%), 3.46 g of magnesium sulfate heptahydrate, 138.2 mg of manganese sulfate pentahydrate, 138.2 mg of ferrous sulfate heptahydrate, 9.14 g of 10N potassium hydroxide aqueous solution, and 105 mL of distilled water were heat-sterilized at 121°C for 20 minutes. Then, considering the amount of evaporation due to the heat sterilization, sterilized water was added to give 320 mL of the total solution. Next, 24 mL of the obtained solution, 55.7 mL of 720 g/L glucose aqueous solution, 80 mL of sterilized water, 545 µL of a pre-filter sterilized vitamin solution (a D-biotin and thiamine hydrochloride (each 0.2 g/L) aqueous solution), 100 mL of 850 mM disodium succinate aqueous solution, and 240 mL of the above-described main culture solution were placed in a 1-L jar fermentor. The total amount of the aqueous medium at this time is 500 mL, and the concentration of the succinic acid alkali metal salt (disodium succinate) in the aqueous medium is 170 mM.

The point in time when all the solutions and the like described above were placed in the 1-L jar fermentor was regarded as the start of reaction. Then, the aqueous medium was stirred at 200 rpm while maintaining the pH of the aqueous medium at 7.6 by adding an ammonia neutralizer (106.5 g of ammonium hydrogen carbonate, 221. 5 g of 18.3N ammonia water, and 704.1 g of sterilized water) and maintaining the temperature thereof at 39°C.

After the elapse of 9.4 hours from the start of the reaction, glucose was almost consumed and the reaction terminated. Table 1 shows a succinic acid concentration at the time of the reaction termination and a succinic acid production rate. The succinic acid production rate is represented by an average value during the reaction time from the start of the reaction to the termination thereof.

### [Comparative Example 1]

Succinic acid production reaction was performed in the same manner as Example 1 except that the 850 mM disodium succinate aqueous solution added to the aqueous medium in (C) the succinic acid production reaction was changed to an 850 mM diammonium succinate aqueous solution. After the elapse of 14.8 hours from the start of the reaction, glucose was almost consumed and the reaction terminated. Table 1 shows the results of the items mentioned above.

### [Comparative Example 2]

Succinic acid production reaction was performed in the same manner as Example 1 except that the ammonia neutralizer used for the pH adjustment of the aqueous medium in (C) the succinic acid production reaction was changed to a sodium neutralizer (113.5 g of ammonium hydrogen carbonate, 145.9 g of sodium hydroxide, and 936.7 g of sterilized water). After the elapse of 12.0 hours from the start of the reaction, glucose was almost consumed and the reaction terminated. Table 1 shows the results of the items mentioned above.

**Table 1**

| | Kind of neutralizer | Disodium succinate concentration at start of reaction [mM] | Succinic acid concentration at start of reaction [g/L] | Succinic acid concentration at termination of reaction [g/L] | Reaction time [h] | Succinic acid production rate [g/L/h] |
|---|---|---|---|---|---|---|
| Example 1 | Ammonia neutralizer | 170 | 20.0 | 57.6 | 9.4 | 4.00 |
| Comparative Example 1 | Ammonia neutralizer | 0 | 20.0 | 57.9 | 14.8 | 2.56 |
| Comparative Example 2 | Sodium neutralizer | 170 | 20.0 | 61.0 | 12.0 | 3.42 |

Table 1 shows that Example 1 improved the succinic acid production rate by 56% as compared to Comparative Example 1 and by 17% as compared to Comparative Example 2.

The results above demonstrated that the condition of adding the ammonia neutralizer in the presence of 170 mM disodium succinate improved the succinic acid production rate as compared to the condition of adding the ammonia neutralizer in the presence of 170 mM diammonium succinate and the condition of adding the sodium neutralizer in the presence of 170 mM disodium succinate.

### [Example 2]

(A) The seed culture and the (B) main culture were performed in the same conditions as Example 1.

### (C) Succinic Acid Production Reaction

An amount of 5.2 g of a phosphoric acid aqueous solution (85 wt%), 3.46 g of magnesium sulfate heptahydrate, 138.2 mg of manganese sulfate pentahydrate, 138.2 mg of ferrous sulfate heptahydrate, 9.14 g of 10N potassium hydroxide aqueous solution, and 105 mL of distilled water were heat-sterilized at 121°C for 20 minutes. Then, considering the amount of evaporation due to the heat sterilization, sterilized water was added to give 320 mL of the total solution.

An amount of 407 mL of the main culture solution was centrifuged (8000 rpm, 10 minutes, 20°C), and the supernatant (357 mL) was discarded. To bacterial cells obtained by the centrifugation were added 24 mL of 320 mL of the solution mentioned above, 170.4 mL of sterilized water, and 545 µL of a pre-filter sterilized vitamin solution (a D-biotin and thiamine hydrochloride (each 0.2 g/L) aqueous solution) to obtain a suspension of the bacterial cells. The total amount of the suspension, 55.7 mL of 720g/L glucose aqueous solution, and 200 mL of 210 mM disodium succinate were placed in a 1-L jar fermentor. The total amount of the aqueous medium at this time is 500 mL, and the concentration of the succinic acid alkali metal salt (disodium succinate) in the aqueous medium is 84 mM.

The point in time when all the solutions and the like described above were placed in the 1-L jar fermentor was regarded as the start of the reaction, and then, the aqueous medium was stirred at 200 rpm while maintaining the pH of the aqueous medium at 7.6 by adding an ammonia neutralizer (106.5 g of ammonium hydrogen carbonate, 221. 5 g of 18.3N ammonia water, and 704.1 g of sterilized water) and maintaining the temperature thereof at 39°C.

After the elapse of 6.3 hours from the start of the reaction, glucose was almost consumed and the reaction terminated. Table 2 shows a succinic acid concentration at the time of the reaction termination and a succinic acid production rate. The succinic acid production rate is represented by an average value during the reaction time from the start of the reaction to the termination thereof.

### [Comparative Example 3]

Succinic acid production reaction was performed in the same manner as Example 2 except that the ammonia neutralizer used for the pH adjustment of the reaction solution in (C) the succinic acid production reaction was changed to a sodium neutralizer (113.5 g of sodium hydrogen carbonate, 145.9 g of sodium hydroxide, and 936.7 g of sterilized water). After the elapse of 8.6 hours from the start of the reaction, glucose was almost consumed and the reaction terminated. Table 2 shows the results of the items mentioned above.

**Table 2**

| | Kind of neutralizer | Disodium succinate concentration at start of reaction [mM] | Succinic acid concentration at start of reaction [g/L] | Succinic acid concentration at termination of reaction [g/L] | Reaction time [h] | Succinic acid production rate [g/L/h] |
|---|---|---|---|---|---|---|
| Example 2 | Ammonia neutralizer | 84 | 10.0 | 48.5 | 6.3 | 6.11 |
| Comparative Example 3 | Sodium neutralizer | 84 | 10.0 | 58.5 | 8.6 | 5.64 |

Table 2 shows that Example 2 improved the succinic acid production rate by 8% as compared to Comparative Example 3.

The results above demonstrated that the condition of adding the ammonia neutralizer in the presence of 84 mM disodium succinate improved the succinic acid production rate as compared to the condition of adding the sodium neutralizer in the presence of 84 mM disodium succinate.

[Example 3]

### <Succinic Acid Production by Changing from Sodium Neutralization to Ammonia Neutralization>

(A) The seed culture and (B) the main culture were performed in the same manner as Example 2.

### (C) Succinic Acid Production Reaction

Succinic acid production reaction was performed as follows. An amount of 5.2 g of a phosphoric acid aqueous solution (85 wt%), 3.46 g of magnesium sulfate heptahydrate, 138.2 mg of manganese sulfate pentahydrate, 138.2 mg of ferrous sulfate heptahydrate, 9.14 g of 10N potassium hydroxide aqueous solution, and 105 mL of distilled water were heat-sterilized at 121°C for 20 minutes. Then, considering the amount of evaporation due to the heat sterilization, sterilized water was added to give 320 mL of the total solution.

Next, 20 mL of 320 mL of the above solution, 168 mL of sterilized water, 448 µL of a pre-filter sterilized vitamin solution (a D-biotin and thiamine hydrochloride (each 0.2 g/L) aqueous solution), 86.5 mL of 720 g/L glucose aqueous solution, and 175 mL of the main culture solution were placed in a 1-L jar fermentor.

The point in time when all the solutions and the like described above were placed in the 1 L jar fermentor was regarded as the start of the reaction. Then, the aqueous medium was stirred at 200 rpm while maintaining the pH of the aqueous medium at 7.6 by adding a sodium neutralizer (113.5 g of sodium hydrogen carbonate, 145.9 g of sodium hydroxide, and 936.7 g of sterilized water) and maintaining the temperature thereof at 39°C.

The amount of the sodium neutralizer added up to the elapse of 3.9 hours from the start of the reaction was 54.7 g. The concentration of succinic acid measured at this time was 20.7 g/L, and the concentration of disodium succinate was 175 mM.

At this point in time, the sodium neutralizer was changed to an ammonia neutralizer (106.5 g of ammonium hydrogen carbonate, 221.5 g of 13.8 N ammonia water, and 704.1 g of sterilized water).

The concentration of succinic acid measured at the time of the elapse of 5.0 hours from the start of the reaction was 28.8 g/L. Table 3 shows a succinic acid production rate represented by an average value during a reaction time from the changing of the neutralizer to the measurement of the succinic acid concentration.

### [Example 4]

Succinic acid production reaction was performed in the same manner as Example 3 except that the changing of the sodium neutralizer to the ammonium neutralizer in (C) the succinic acid production reaction was performed after the elapse of 5.0 hours from the start of the reaction.

The amount of the sodium neutralizer added up to this point in time was 80.2 g, and the concentrations of succinic acid and disodium succinate, respectively, measured at this time were 27.6 g/L and 234 mM, respectively.

The concentration of succinic acid measured after the elapse of 6.1 hours from the start of the reaction was 36.2 g/L. Table 4 shows a succinic acid production rate represented by an average value during a reaction time from the changing of the neutralizer to the measurement of the succinic acid concentration.

### [Example 5]

Succinic acid production reaction was performed in the same manner as Example 3 except that the changing of the sodium neutralizer to the ammonium neutralizer in (C) the succinic acid production reaction was performed after the elapse of 6.1 hours from the start of the reaction.

The amount of the sodium neutralizer added up to this point in time was 106.1 g, and the concentrations of succinic acid and disodium succinate, respectively, measured at this time were 35.5 g/L and 301 mM, respectively.

The concentration of succinic acid measured after the elapse of 8.2 hours from the start of the reaction was 47.7 g/L. Table 5 shows a succinic acid production rate represented by an average value during a reaction time from the changing of the neutralizer to the measurement of the succinic acid concentration.

### [Comparative Example 4]

Succinic acid production reaction was performed in the same manner as Example 3 except that the sodium neutralizer first used for the pH adjustment of the aqueous medium in (C) the succinic acid production reaction was changed to an ammonia neutralizer (106.5 g of ammonium hydrogen carbonate, 221.5 g of 13.8 N ammonia water, and 704.1 g of sterilized water) and there was no changing of the neutralizer after that.

Succinic acid concentrations measured at the times of the elapses of 2.8 hours, 3.9 hours (corresponding to Comparative Example 6), 5.0 hours (corresponding to Example 3), 6.1 hours (corresponding to Example 4), and 8.2 hours (corresponding to Example 5), respectively, from the start of the reaction were 11.0 g/L, 21.7 g/L, 28.6 g/L, 34.9 g/L, and 43.5 g/L, respectively.

Tables 3, 4, 5, and 6, respectively, show succinic acid production rates as the results of Comparative Example 4, which are corresponding to Examples, 3, 4, and 5 and Comparative Example 6.

In Tables 3, 4, 5, and 6, the succinic acid production rates (g/L/h) are those calculated from the succinic acid concentrations (g/L) measured at the two points in time in each of the Tables.

### [Comparative Example 5]

Succinic acid production reaction was performed in the same manner as Example 3 except for not changing the neutralizer. Succinic acid concentrations measured at the times of the elapses of 2.8 hours, 3.9 hours (corresponding to Comparative Example 6), 5.0 hours (corresponding to Example 3), 6.1 hours (corresponding to Example 4), and 8.2 hours (corresponding to Example 5), respectively, from the start of the reaction were 8.8 g/L, 20.0 g/L, 27.7 g/L, 35.3 g/L, and 45.8 g/L, respectively.

Tables 3, 4, 5, and 6, respectively, show succinic acid production rates as the results of Comparative Example 5, which are corresponding to Examples, 3, 4, and 5 and Comparative Example 6.

### [Comparative Example 6]

Succinic acid production reaction was performed in the same manner as Example 3 except for changing the neutralizer after the elapse of 2.8 hours from the start of the reaction. The amount of the sodium neutralizer added up to this point in time was 20.4 g, and the concentration of succinic acid measured at this time was 8.9 g/L (the concentration of disodium succinate was 75 mM).

The concentration of succinic acid measured at the time of the elapse of 3.9 hours from the start of the reaction was 19.1 g/L. Table 6 shows a succinic acid production rate represented by an average value during a reaction time from the changing of the neutralizer to the measurement of the succinic acid concentration.

**Table 3**

| | Kind of neutralizer | Disodium succinate concentration after 3.9 hours [mM] | Succinic acid concentration after 3.9 hours [g/L] | Succinic acid concentration after 5.0 hours [g/L] | Reaction time [h] | Succinic acid production rate [g/L/h] |
|---|---|---|---|---|---|---|
| Example 3 | Sodium neutralizer ⇒ Ammonia neutralizer | 175 | 20.7 | 28.8 | 1.1 | 7.31 |
| Comparative Example 4 | Ammonia neutralizer | 0 | 21.7 | 28.6 | 1,1 | 6.20 |
| Comparative Example 5 | Sodium neutralizer | 169 | 20.0 | 27.7 | 1.1 | 7.03 |

Table 3 shows that Example 3 improved the succinic acid production rate by 18% as compared to Comparative Example 4 and by 4% as compared to Comparative Example 5.

The above results demonstrated that the condition of using the sodium neutralizer until the accumulation of disodium succinate of 175 mM and then changing to the ammonia neutralizer improved the succinic acid production rate as compared to the condition of using the ammonia neutralizer alone and the condition of using the sodium neutralizer alone.

**Table 4**

| | Kind of neutralizer | Disodium succinate concentration after 5.0 hours [mM] | Succinic acid concentration after 5.0 hours [g/L] | Succinic acid concentration after 6.1 hours [g/L] | Reaction time [h] | Succinic acid production rate [g/L/h] |
|---|---|---|---|---|---|---|
| Example 4 | Sodium neutralizer ⇒ Ammonia neutralizer | 234 | 27.6 | 36.2 | 1.1 | 7.79 |
| Comparative Example 4 | Ammonia neutralizer | 0 | 28.6 | 34.9 | 1.1 | 5.79 |
| Comparative Example 5 | Sodium neutralizer | 234 | 27.7 | 35.3 | 1.1 | 6.91 |

Table 4 shows that Example 4 improved the succinic acid production rate by 35% as compared to Comparative Example 4 and by 13% as compared to Comparative Example 5.

The above results demonstrated that the condition of using the sodium neutralizer until the accumulation of disodium succinate of 234 mM and then changing to the ammonia neutralizer improved the succinic acid production rate as compared to the condition of using the ammonia neutralizer alone and the condition of using the sodium neutralizer alone.

**Table 5**

| | Kind of neutralizer | Disodium succinate concentration after 6.1 hours [mM] | Succinic acid concentration after 6.1 hours [g/L] | Succinic acid concentration after 8.2 hours [g/L] | Reaction time [h] | Succinic acid production rate [g/L/h] |
|---|---|---|---|---|---|---|
| Example 5 | Sodium neutralizer ⇒ Ammonia neutralizer | 301 | 35.5 | 47.7 | 2.1 | 5.78 |
| Comparative Example 4 | Ammonia neutralizer | 0 | 34.9 | 43.5 | 2.1 | 4.07 |
| Comparative Example 5 | Sodium neutralizer | 299 | 35.3 | 45.8 | 2.1 | 5.01 |

Table 5 shows that Example 5 improved the succinic acid production rate by 42% as compared to Comparative Example 4 and by 15% as compared to Comparative Example 5.

The above results demonstrated that the condition of using the sodium neutralizer until the accumulation of disodium succinate of 301 mM and then changing to the ammonia neutralizer improved the succinic acid production rate as compared to the condition of using the ammonia neutralizer alone and the condition of using the sodium neutralizer alone.

**Table 6**

| | Kind of neutralizer | Disodium succinate concentration after 2.8 hours [mM] | Succinic acid concentration after 2.8 hours [g/L] | Succinic acid concentration after 3.9 hours [g/L] | Reaction time [h] | Succinic acid production rate [g/L/h] |
|---|---|---|---|---|---|---|
| Comparative Example 6 | Sodium neutralizer ⇒ Ammonia neutralizer | 75 | 8.9 | 19.1 | 1.1 | 9.33 |
| Comparative Example 4 | Ammonia neutralizer | 0 | 11.0 | 21.7 | 1.1 | 9.75 |
| Comparative Example 5 | Sodium neutralizer | 75 | 8.8 | 20.0 | 1.1 | 10.17 |

Table 6 shows that Comparative Example 6 reduced the succinic acid production rate by 4% as compared to Comparative Example 4 and by 8% as compared to Comparative Example 5.

The above results demonstrated that the condition of using the sodium neutralizer until the accumulation of disodium succinate of 75 mM and then changing to the ammonia neutralizer reduced the succinic acid production rate as compared to the condition of using the ammonia neutralizer alone and the condition of using the sodium neutralizer alone.

The results of Examples 3 to 5 and Comparative Example 6 are summarized as follows:

**Table 7**

| | Sodium succinate at addition of ammonia (mM) | Improvement rate of succinic acid production rate (%) (With respect to Comparative Example 4) |
|---|---|---|
| Example 3 | 180 | 18 |
| Example 4 | 230 | 35 |
| Example 5 | 300 | 42 |
| Comparative Example 6 | 75 | -4 |

**Table 8**

| | Sodium succinate at addition of ammonia (mM) | Improvement rate of succinic acid production rate (%) (With respect to Comparative Example 5) |
|---|---|---|
| Example 3 | 180 | 5 |
| Example 4 | 230 | 13 |
| Example 5 | 300 | 15 |
| Comparative Example 6 | 75 | -8 |

### [Example 6]

### <Creation of SDH Genome-Enhanced Strain>

### (A) Extraction of MJ233 Strain Genome DNA

A *Brevibacterium flavum* MJ-233 strain was cultured until a late logarithmic growth phase in 10 mL of a culture medium A [2 g of urea, 7 g of (HN₄₎₂SO₄, 0.5 g of KH₂PO₄, 0.5 g of K₂HPO₄, 0.5 g of MgSO₄·7H₂O, 6 mg of FeSO₄·7H₂O, 6mg of MnSO₄·4-5H₂O, 200µg of biotin, 100 µg of thiamine, 1g of yeast extract, 1g of casamino acid, and 20 g of glucose dissolved in 1 L of distilled water], and then centrifuged (10000 g, 5 minutes) to collect bacterial cells. The obtained bacterial cells were suspended in 0.15 mL of a 10 mM NaCl/20 mM Tris buffer solution (pH 8.0)/1 mM EDTA·2Na solution containing lysozyme at a concentration of 10 mg/mL. Next, proteinase K was added to the suspension to give a final concentration of 100 µg/mL, and then the suspension was incubated at 37°C for 1 hour. Furthermore, sodium dodecyl sulfate was added to give a final concentration of 0.5%, and then the suspension was incubated at 50°C for 6 hours to perform bacteriolysis. To the lysate was added an equal amount of a phenol/chloroform solution, and the resulting solution was gently shaken for 10 minutes at room temperature. Then, the total amount of the solution was centrifuged (5,000 xg, 20 minutes, 10 to 12°C), and the supernatant fraction was collected. Next, after adding sodium acetate to a concentration of 0.3 M, a double amount of ethanol was added and mixed. A precipitate recovered by centrifugation (15,000 xg, 2 minutes) was washed with 70% ethanol and air-dried. To the obtained DNA was added 5 mL of a 10 mM Tris buffer (pH 7.5)/1mM EDTA·2Na solution, and the obtained solution was allowed to stand at 4°C overnight. The resulting DNA was used as a template DNA of PCR performed later.

### (B) Construction of Plasmid for Substitution of SDH Promoter An SDH gene of MJ233 strain was obtained by performing PCR using the

DNA prepared in the above (A) as a template and synthetic DNAs (SEQ ID NO: 1 and SEQ ID NO: 2) designed based on the nucleotide sequence of the gene of *Corynebacterium glutamicum* ATCC13032 strain (GenBank Database Accession No. AP005276), for which the complete genome sequence has been known. Reaction solution composition: 1 µL of the template DNA, 0.2 µL of PfxDNA polymerase (manufactured by Invitrogen Co. Ltd.), 1-fold concentration of attached buffer, 0.3 µM of each primer, 1 mM of MgSO₄, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 50 seconds was repeated 35 times, where exceptionally, retention time at 94°C in the first cycle was 1 minute and 20 seconds, and retention time at 72°C in the final cycle was 5 minutes.. The amplification product was confirmed by separation using gel electrophoresis on a 0.75% agarose (SeaKem GTG agarose: manufactured by FMC BioProducts Ltd.) and then visualization by ethidium bromide staining, whereby a DNA fragment of approximately 0.8 kb was detected. The target DNA fragment was recovered from the gel using a QIA Quick Gel Extraction Kit (manufactured by Quiagen Inc). After phosphorylating the 5'-terminal of the recovered DNA fragment using T4 polynucleotide kinase (manufactured by Takara Shuzo Co. Ltd.), the fragment was ligated to the HincII site of an *Escherichia coli* vector pHSG299 (manufactured by Takara Shuzo Co., Ltd.) using a ligation kit ver. 2 (manufactured by Takara Shuzo Co., Ltd), and then, *Escherichia coli* (DH5α strain) was transformed using the obtained plasmid DNA. The recombinant *Escherichia coli* thus obtained was smeared onto an LB agar medium containing 50 µg/ml of kanamycin and 50 µg/mL, of X-Gal. Clones forming white colonies on the medium were liquid cultured by a usual method, and then plasmid DNA was purified. The obtained plasmid DNA was subjected to PCR reaction using the synthetic DNAs represented by SEQ ID NOs: 1 and 2 as primers. Reaction solution composition: 1 ng of the above plasmid, 0.2 µL of Ex-TaqDNA polymerase (manufactured by Takara Shuzo Co., Ltd.), 1-fold concentration of attached buffer, 0.2 µM of each primer, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 50 seconds was repeated 20 times, where exceptionally, retention time at 94°C in the first cycle was 1 minute and 20 seconds, and retention time at 72°C in the final cycle was 5 minutes. As a result of confirming the presence or absence of the inserted DNA fragment in this manner, there was selected a plasmid in which the amplification product of approximately 0.8 kb was observed. The plasmid was named pSDH764.

Next, PCR was performed using the plasmid DNA pSDH764 as a template and synthetic DNAs (SEQ ID NO: 3 and SEQ ID NO: 4) designed based on the nucleotide sequence of the gene of the *Corynebacterium glutamicum* ATCC13032 strain (GenBank Database Accession No. AP005276), for which the complete genome sequence has been known. The DNA of SEQ ID NO: 3 used was a 5'-phosphorylated DNA. Reaction solution composition: 1 µL of the template DNA, 0.2 µL of PfxDNA polymerase (manufactured by Invitrogen Co. Ltd.), 1-fold concentration of attached buffer, 0.3 µM of each primer, 1 mM of MgSO₄, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 50 seconds was repeated 20 times, where exceptionally, retention time at 94°C in the first cycle was 1 minute and 20 seconds, and retention time at 72°C in the final cycle was 4 minutes.. The amplification product was confirmed by separation using gel electrophoresis on a 0.75% agarose (SeaKem GTG agarose: manufactured by FMC BioProducts Ltd.) and then visualization by ethidium bromide staining, whereby a DNA fragment of approximately 0.7 kb was detected. The target DNA fragment was recovered from the gel using a QIA Quick Gel Extraction Kit (manufactured by Quiagen Inc.), and used as an SDH gene N'-terminal segment.

A TZ4 promoter fragment derived from *Brevibacteriumflavum* MJ233 strain and constitutively highly expressed was prepared by PCR using a plasmid pMJPC1 (JP-A-2005-95169) as a template and synthetic DNAs represented by SEQ ID NOs: 5 and 6. The DNA of SEQ ID NO: 6 used was a 5'-phospholylated DNA. Reaction solution composition: 1 µL of the template DNA, 0.2 µL of PfxDNA polymerase (manufactured by Invitrogen Co. Ltd.), 1-fold concentration of attached buffer, 0.3 µM of each primer, 1 mM of MgSO₄, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 30 seconds was repeated 25 times, where exceptionally, retention time at 94°C in the first cycle was 1 minute and 20 seconds, and retention time at 72°C in the final cycle was 3 minutes. The confirmation of the amplification product was made by separation using gel electrophoresis on a 1.0% agarose (SeaKem GTG agarose: manufactured by FMC BioProducts Ltd.) and then visualization by ethidium bromide staining, whereby a DNA fragment of approximately 0.5 kb was detected. The target DNA fragment was recovered from the gel using a QIA Quick Gel Extraction Kit (manufactured by Quiagen Inc.), and used as the TZ4 promoter fragment.

The SDH gene N'-terminal fragment and the TZ4 promoter fragment prepared above were mixed and then ligated with each other by a ligation kit ver. 2 (manufactured by Takara Shuzo Co. Ltd). Then, the resulting fragment was digested with restriction enzymes KpnI and SphI and separated by gel electrophoresis on a 0.75% agarose (SeaKem GTG agarose: manufactured by FMC BioProducts Ltd.), whereby a DNA fragment of approximately 0.95 kb was recovered using a QIA Quick Gel Extraction Kit (manufactured by Quiagen Inc.) and defined as a TZ4 promoter::SDH gene N'-terminal fragment. Additionally, the DNA fragment was mixed with a DNA prepared by digesting an *Escherichia coli* plasmid pHSG299 (manufactured by Takara Shuzo Co., Ltd.) with restriction enzymes KpnI and SphI and then ligated thereto using a ligation kit ver. 2 (manufactured by Takara Shuzo Co. Ltd). Then, *Escherichia coli* (DH5α strain) was transformed using the obtained plasmid DNA. The recombinant *Escherichia coli* thus obtained was smeared onto an LB agar medium containing 50 µg/ml of kanamycin and 50 µg/mL of X-Gal. Clones forming white colonies on the medium were liquid-cultured by a usual method, and then plasmid DNA was purified. The obtained plasmid DNA was digested with restriction enzymes KpnI and SphI, whereby the inserted fragment of approximately 0.95 kb was observed and the plasmid was named SDH15.1.

A DNA fragment of a 5' upstream region of the *Brevibacterium flavum* MJ233-derived SDH gene was obtained by PCR using the DNA prepared in Example 6 (A) as a template and synthetic DNAs (SEQ ID NO: 7 and SEQ ID NO: 8) designed based on the nucleotide sequence of the gene of the *Corynebacterium glutamicum* ATCC13032 strain (GenBank Database Accession No. AP005276), for which the complete genome sequence has been known. Reaction solution composition: 1 µL of the template DNA, 0.2 µL of PfxDNA polymerase (manufactured by Invitrogen Co. Ltd.), 1-fold concentration of attached buffer, 0.3 µM of each primer, 1 mM of MgSO₄, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 40 seconds was repeated 35 times, where exceptionally, retention time at 94°C in the first cycle was 1 minute and 20 seconds, and retention time at 72°C in the final cycle was 5 minutes. The amplification product was confirmed by separation using gel electrophoresis on a 1.0% agarose (SeaKem GTG agarose: manufactured by FMC BioProducts Ltd.) and then visualization by ethidium bromide staining, whereby a fragment of approximately 0.6 kb was detected. The target DNA fragment was recovered from the gel using a QIA Quick Gel Extraction Kit (manufactured by Quiagen Inc). The recovered DNA fragment was phosphorylated at the 5' terminal by T4 polynucleotide kinase (manufactured by Takara Shuzo Co. Ltd.), and then ligated to the EcoRV site of an *Escherichia coli* vector pBluescriptII (manufactured by Strategene Co. Ltd.) using a ligation kit ver. 2 (manufactured by Takara Shuzo Co., Ltd). The obtained plasmid DNA was used to transform *Escherichia coli* (DH5α strain). The recombinant *Escherichia coli* thus obtained was smeared onto an LB agar medium containing 50 µg/ml of ampicillin and 50 µg/mL of X-Gal. Clones forming white colonies on the medium were liquid-cultured by a usual method, and then plasmid DNA was purified. The obtained plasmid DNA was subjected to PCR reaction using the synthetic DNAs represented by SEQ ID NOs: 7 and 8 as primers. Reaction solution composition: 1 ng of the above plasmid, 0.2 µL of Ex-TaqDNA polymerase (manufactured by Takara Shuzo Co., Ltd.), 1-fold concentration of attached buffer, 0.2 µM of each primer, and 0.25 µM of dNTPs were mixed together to give a total amount of 20 µL. Reaction temperature conditions: using a DNA thermal cycler PTC-200 (manufactured by MJ Research Inc.), one cycle consisting of 94°C for 20 seconds, 60°C for 20 seconds, and 72°C for 40 seconds was repeated 20 times, where exceptionally, retention time at 94°C in the first cycle was 1 minute and 20 seconds, and retention time at 72°C in the final cycle was 5 minutes. As a result of confirming the presence or absence of the inserted DNA fragment in this manner, there was selected a plasmid in which the amplification product of approximately 0.6 kb was observed. The plasmid was named pSDH5.1.

Next, the SDH15.1 and the pSDH5.1, respectively, digested with a restriction enzyme KpnI, were mixed with each other and then ligated using a ligation kit ver. 2 (manufactured by Takara Shuzo Co., Ltd). The obtained product was digested with restriction enzymes SacI and SphI to obtain a DNA fragment, which was then separated by gel electrophoresis on a 0.75% agarose (SeaKem GTG agarose: manufactured by FMC BioProducts Ltd.), resulting in the recovery of a DNA fragment of approximately 1.7 kb using a QIA Quick Gel Extraction Kit (manufactured by Quiagen Inc). The DNA fragment having the TZ4 promoter inserted between the 5' upstream region and the N' terminal region of the SDH gene was mixed with a DNA prepared by digesting pKMB1 (JP-A-2005-95169) with SacI and SphI, and both DNAs were ligated to each other using a ligation kit ver. 2 (manufactured by Takara Shuzo Co. Ltd). The obtained plasmid DNA was used to transform *Escherichia coli* (DH5α strain). The recombinant *Escherichia coli* thus obtained was smeared onto an LB agar medium containing 50 µg/ml of kanamycin and 50 µg/mL of X-Gal. Clones forming white colonies on the medium were liquid-cultured by a usual method, and then plasmid DNA was purified. The obtained plasmid DNA was digested with restriction enzymes SacI and SphI, whereby the inserted fragment of approximately 1.7 kb was observed, and the plasmid was named pSDH15.2.

### (C) Creation of SDH Promoter-Substituted Strain

A plasmid DNA used for transformation of the *Brevibacterium flavum* MJ233/PC-4/ΔLDH strain (WO 2009/025363) was reprepared from *Escherichia coli* JM110 strain transformed using the plasmid DNA pSDH15.2 by a calcium chloride method (Journal of Molecular Biology, 53, 159, 1970). The transformation of the *Brevibacterium flavum* MJ233/PC-4/ΔLDH strain was performed by an electric pulse method (Res. Microbiol., 1993 Vol. 144: 181-5), and the obtained transformant was smeared onto an LBG agar medium containing 50 µg/mL of kanamycin [10 g of tryptone, 5 g of yeast extract, 5 g of NaCl, 20 g of glucose, and 15 g of agar, dissolved in 1 L of distilled water]. Since the pSDH15.2 is a plasmid not replicable in bacterial cells of *Brevibacterium flavum* MJ233, it is expected in the strain grown on this medium that as a result of causing homologous recombination between the SDH gene of the plasmid and the SDH gene on the *Brevibacterium flavum* MJ233 strain genome, a kanamycin-resistant gene and a SacB gene derived from the plasmid have been inserted in the strain genome. Next, the homologous recombinant strain was liquid-cultured in an LBG medium containing 50 µg/mL of kanamycin. Bacterial cells of the culture solution were smeared in an amount corresponding to approximately 1 million cells onto a 10% sucrose-containing LBG medium, as a result of which there were obtained several tens of strains that seemed to have become sucrose-insensitive due to the loss of the SacB gene by the second homologous recombination. The strains thus obtained include those having an SDH gene in which the pSDH15.2-derived TZ4 promoter has been inserted and those having an SDH gene converted back to a wild type. Whether the SDH gene is a mutant type or a wild type can be easily determined by directly performing PCR reaction on bacterial cells obtained by liquid culture in an LBG medium to detect SDH gene. Analysis using primers (SEQ ID NOs: 9 and 2) for PCR amplification of the TZ4 promoter and the SDH gene should show a DNA fragment of 748 bp in the mutant type. As a result of analysis of the sucrose-insensitive bacterial strains by the above method, a TZ4 promoter-inserted strain was selected and named *Brevibacterium flavum* MJ233/SDH/PC-4/ΔLDH.

### (D) Measurement of Fumarate Reductase Enzyme Activity

The transformed strain *Brevibacterium flavum* MJ233/SDH/PC-4/ΔLDH obtained in the above (C) was cultured overnight in 100 ml of culture medium A containing 2% glucose. The obtained bacterial cells were collected, washed with 50 mL of 50 mM potassium phosphate buffer (pH 7.5), and then suspended again in 20 mL of buffer solution having the same composition. The resulting suspension was crushed by SONIFIER 350 (manufactured by Branson) and centrifuged to obtain a supernatant as a cell-free extract. The obtained cell-free extract was used to measure FRD activity. The measurement of the enzyme activity was performed by reacting the extract at 25°C in a reaction solution containing 33 mM Tris/HCl buffer solution (pH 7.5), 0.1 mM of EDTA, 20 mM of disodium succinate, 2 mM of K₃Fe(CN)₆, and the enzyme. 1U was defined as an amount of the enzyme for catalyzing a reduction of K₃Fe(CN)₆ of 2 µmol per minute. The cell-free extract of the *Brevibacteriumflavum* MJ233/SDH/PC-4/ΔLDH strain had an FRD specific activity of 0.02 U/mg-protein. In addition, in bacterial cells obtained by culturing the parent strain MJ233/PC-4/ΔLDH in the same manner, the FRD specific activity was 0.01 U/mg-protein. Accordingly, it was shown that the substitution of the TZ4 promoter increased the FRD activity of the *Brevibacterium flavum* MJ233/SDH/PC-4/ΔLDH strain to approximately twice the parent strain.

### [Example 7]

### <Creation of MDH Genome-Enhanced Strain>

### (A) Construction of Plasmid for Substitution of MDH Promoter

A malate dehydrogenase gene derived from the *Brevibacterium flavum* MJ233 strain was obtained by PCR using the DNA prepared in the above (A) as a template and synthetic DNAs (SEQ ID NO: 10 and SEQ ID NO: 11) designed based on the nucleotide sequence of the gene of the *Corynebacterium glutamicum* ATCC13032 strain (GenBank Database Accession No. AP005276), for which the complete genome sequence has been known. Reaction solution composition: 1 µL of the template DNA, 0.5 µL of PfxDNA polymerase (manufactured by Invitrogen Co. Ltd.), 1-fold concentration of attached buffer, 0.4 µM of each primer, 1 mM of MgSO₄, and 0.2 µM of dNTPs were mixed together to give a total amount of 50 µL. Reaction temperature conditions: using a DNA thermal cycler PTC-200 (manufactured by MJ Research Inc.), one cycle consisting of 94°C for 10 seconds, 60°C for 20 seconds, and 72°C for 1 minute was repeated 35 times, where exceptionally, retention time at 94°C in the first cycle was 2 minutes, and retention time at 72°C in the final cycle was 4 minutes. After terminating the PCR reaction, 0.1 µL of Takara Ex Taq (Takara Shuzo) was added, and the resulting solution was additionally maintained at 72°C for 30 minutes. The confirmation of the amplification product was performed by separation using gel electrophoresis on a 0.75% agarose (SeaKem GTG agarose: manufactured by FMC BioProducts Ltd.) and then visualization by ethidium bromide staining, whereby a fragment of approximately 1.0 kb was detected. The target DNA fragment was recovered from the gel using a QIA Quick Gel Extraction Kit (manufactured by Quiagen Inc). The recovered DNA fragment was mixed with a PCR product cloning vector pT7Blue T-Vector (manufactured by Novagen) and ligated thereto using a ligation kit ver. 2 (manufactured by Takara Shuzo Co., Ltd). The obtained plasmid DNA was used to transform *Escherichia coli* (DH5α strain). The recombinant *Escherichia coli* thus obtained was smeared onto an LB agar medium containing 50 µg/ml of ampicillin and 50 µg/mL of X-Gal. Clones forming white colonies on the medium were liquid-cultured by a usual method, and then plasmid DNA was purified. The obtained plasmid DNA was digested with restriction enzymes ApaI and PacI, whereby the inserted fragment of approximately 1.0 kb was observed, and the plasmid was named pCoMDH1.

Next, a DNA fragment of approximately 1.0 kb obtained by digesting the pCoMDH1 with restriction enzymes ApaI and PacI was separated and recovered by 0.75% agarose gel electrophoresis, and then mixed with a DNA prepared by digesting the plasmid pMJPC1 (JP-A-2005-95169) with restriction enzymes ApaI and PacI. Both DNAs were ligated to each other using a ligation kit ver. 2 (manufactured by Takara Shuzo Co. Ltd). Using the plasmid DNA thus obtained, *Escherichia coli* (DH5α strain) was transformed, and then smeared onto an LB agar medium containing 50 µg/ml of kanamycin. Colonies grown on the medium were liquid-cultured by a usual method, and then plasmid DNA was purified. The obtained plasmid DNA was digested with restriction enzymes ApaI and PacI, whereby there was selected a plasmid containing the inserted fragment of approximately 1.0 kb and the plasmid was named pMDH37.

Next, the pMDH37 was digested with a restriction enzyme HindIII, and the section thereof was smoothed by Klenow Fragment (manufactured by Takara Shuzo Co. Ltd) and digested with a restriction enzyme XbaI. A DNA fragment of approximately 0.9 kb thus obtained was detected by separation using gel electrophoresis on a 0.8% agarose (SeaKem GTG agarose: manufactured by FMC BioProducts Ltd.) and then visualization by ethidium bromide staining, and recovered from the gel using a Gel Extraction System (manufactured by Maligen). The DNA fragment containing the TZ4 promoter and the N-terminal region of the MDH gene was mixed with a DNA prepared by digesting the pKMB1 (JP-A-2005-95169) with a restriction enzyme BamHI, smoothing the section by Klenow Fragment (manufactured by Takara Shuzo Co. Ltd.), and then digesting with a restriction enzyme XbaI and ligated thereto using a ligation kit ver. 2 (manufactured by Takara Shuzo Co., Ltd). The obtained plasmid DNA was used to transform *Escherichia coli* (DH5α strain), and the transformant was smeared on an LB agar medium containing 50 µg/ml of kanamycin and 50 µg/mL of X-Gal. Clones forming white colonies on the medium were liquid-cultured by a usual method, and then plasmid DNA was purified. The obtained plasmid DNA was digested with restriction enzymes SalI and BglII, whereby a part of the inserted fragment of approximately 0.7 kb was observed, and the plasmid was named pKB-MDH1.

A DNA fragment of a 5' upstream region of the *Brevibacterium flavum* MJ233-derived MDH gene was obtained by PCR using the DNA prepared in Example 6 (A) as a template and synthetic DNAs (SEQ ID NO: 12 and SEQ ID NO: 13) designed based on the nucleotide sequence of the gene of the *Corynebacterium glutamicum* ATCC13032 strain (GenBank Database Accession No. AP005276), for which the complete genome sequence has been known. The synthetic DNAs contain the recognition sequence of restriction enzyme SalI and XbaI, respectively. Reaction solution composition: 1 µL of the template DNA, 0.5 µL of PfxDNA polymerase (manufactured by Invitrogen Co. Ltd.), 1-fold concentration of attached buffer, 0.4 µM of each primer, 1 mM of MgSO₄, and 0.2 µM of dNTPs were mixed together to give a total amount of 50 µL. Reaction temperature conditions: using a DNA thermal cycler PTC-200 (manufactured by MJ Research Inc.), one cycle consisting of 94°C for 15 seconds, 55°C for 30 seconds, and 68°C for 45 seconds was repeated 35 times, where exceptionally, retention time at 94°C in the first cycle was 2 minutes, and retention time at 68°C in the final cycle was 3 minutes. The amplification product was purified using a Rapid PCR Purification System (manufactured by Maligen), and then digested with restriction enzymes SalI and XbaI, thereby obtaining a DNA fragment of approximately 0.6 kb. The DNA fragment thus obtained was detected by separation using gel electrophoresis on a 0.8% agarose (SeaKem GTG agarose: manufactured by FMC BioProducts Ltd.) and then visualization by ethidium bromide staining. The DNA fragment was recovered from the gel using a Gel Extraction System (manufactured by Maligen). The recovered DNA fragment was mixed with a DNA prepared by digesting the pKB-MDH1 with restriction enzymes SalI and XbaI and ligated thereto using a ligation kit ver. 2 (manufactured by Takara Shuzo Co., Ltd). The obtained plasmid DNA was used to transform *Escherichia coli* (DH5α strain), and the obtained transformant was smeared on an LB agar medium containing 50 µg/ml of kanamycin and 50 µg/mL of X-Gal. Clones forming white colonies on the medium were liquid-cultured by a usual method, and then plasmid DNA was purified. The obtained plasmid DNA was digested with restriction enzymes SalI and BglII, whereby a fragment of approximately 1.2 kb containing the inserted fragment was observed, and the plasmid was named pKB-MDH2.

### (B) Creation of MDH Promoter-Substituted Strain

A plasmid DNA used for transformation of the *Brevibacterium flavum* MJ233/SDH/PC-4/ΔLDH strain was reprepared from *Escherichia coli* JM110 strain transformed using the above-mentioned plasmid DNA pKB-MDH2 by the calcium chloride method (Journal of Molecular Biology, 53, 159, 1970). The transformation of the *Brevibacterium flavum* MJ233/SDH/PC-4/ΔLDH strain was performed by the electric pulse method shown in the (C) of Example 6, and the obtained transformant was smeared on an LBG agar medium containing 50 µg/mL of kanamycin. Since the pKB-MDH2 is a plasmid not replicable in bacterial cells of *Brevibacterium flavum* MJ233, it is expected in the strain grown on this medium that as a result of causing homologous recombination between the MDH gene of the plasmid and the MDH gene on the *Brevibacterium flavum* MJ233 strain genome, a kanamycin-resistant gene and a SacB gene derived from the plasmid have been inserted in the genome. Next, the homologous recombinant strain was liquid-cultured in an LBG medium containing 50 µg/mL of kanamycin. Bacterial cells of the culture solution were smeared in an amount corresponding to approximately 1 million cells onto a 10% sucrose-containing LBG medium, as a result of which there were obtained several tens of strains that seemed to have become sucrose-insensitive due to the loss of the SacB gene by the second homologous recombination. The strains thus obtained include those having an MDH gene in which the pKB-MDH2-derived TZ4 promoter has been inserted and those having an MDH gene converted back to a wild type. Whether the MDH gene is a mutant type or a wild type can be easily determined by directly performing PCR reaction on bacterial cells obtained by liquid culture in an LBG medium to detect MDH gene. Analysis using primers (SEQ ID NOs: 9 and 14) for PCR amplification of the TZ4 promoter and the MDH gene should show a DNA fragment of 1242 bp in the mutant type. As a result of analysis of the sucrose-insensitive bacterial strains by the above method, a TZ4 promoter-inserted strain was selected and named *Brevibacterium flavum* MJ233/MDH/SDH/PC-4/ΔLDH.

### (C) Measurement of Malate Dehydrogenase Enzyme Activity

The *Brevibacterium flavum* MJ233/MDH/SDH/PC-4/ΔLDH strain obtained in the above (B) was cultured overnight in 100 ml of culture medium A containing 2% glucose. After collecting 10 mL of the obtained culture solution of bacterial cells, the bacterial solution was washed twice with 10 mL of 50 mM Hepes/NaOH buffer solution (pH 7.5) containing 5 mM of MgCl₂ and suspended again in 2 ml of 50 mM Hepes/NaOH buffer solution (pH 7.5) containing 4.5 M of glycerol and 5 mM of MgCl₂. The suspension was crushed with a Bioruptor (manufactured by Cosmo Bio Co., Ltd.) and then centrifuged (10,000 G, 15 minutes). The obtained supernatant was furthermore ultracentrifuged (40,000 rpm, 60 minutes) to obtain a supernatant as a cell-free extract. The obtained cell-free extract was used to measure MDH activity. The measurement of the enzyme activity was performed by reacting the extract at 25°C in a reaction solution containing 100 mM Tris/HCl buffer solution (pH 7.5), 5 mM of MgCl₂, 5 mM of oxaloacetic acid, 0.32 mM ofNADH, and the enzyme. 1 U was defined as an amount of the enzyme for catalyzing a reduction ofNADH of 1 µmol per minute. According to the above measurement method, the *Brevibacterium flavum* MJ233/MDH/SDH/PC-4/ΔLDH strain had an MDH specific activity of 3.73 U/mg-protein. In addition, in bacterial cells obtained by culturing the parent strain *Brevibacterium flavum* MJ233/SDH/PC-4/ΔLDH in the same manner, the MDH specific activity was 1.22 U/mg-protein. Accordingly, it was shown that the substitution of the TZ4 promoter increased the MDH specific activity thereof to approximately three times the parent strain.

### [Example 8]

### <Succinic Acid Production by Changing from Sodium Neutralization to Ammonia Neutralization>

(A) The seed culture and (B) the main culture were performed in the same manner as Example 1 except for using the MJ233/MDH/SDH/PC-4/ΔLDH strain created in Example 7.

### (C) Succinic Acid Production Reaction

Succinic acid production reaction was performed as follows. An amount of 5.2 g of phosphoric acid aqueous solution (85 wt%), 3.46 g of magnesium sulfate heptahydrate, 138.2 mg of manganese sulfate pentahydrate, 138.2 mg of ferrous sulfate heptahydrate, 9.14 g of 10N potassium hydroxide aqueous solution, and 105 mL of distilled water were heat-sterilized at 121°C for 20 minutes, and then sterilized water was added thereto to give 320 mL of the total solution in consideration of the amount of evaporation due to the heat sterilization.

An amount of 20 mL of the 320 mL solution above, 103 mL of sterilized water, 448 µL of a pre-filter sterilized vitamin solution (a D-biotin and thiamine hydrochloride (each 0.2 g/L) aqueous solution), 111.5 mL of 720 g/L glucose aqueous solution, and 175 mL of the main culture solution were placed in a 1-L jar fermentor.

The point in time when all the solutions and the like described above were placed in the 1-L jar fermentor was regarded as the start of the reaction. Then, the aqueous medium was stirred at 200 rpm while maintaining the pH thereof at 7.6 by adding a sodium neutralizer (113.5 g of sodium hydrogen carbonate, 145. 9 g of sodium hydroxide, and 936.7 g of sterilized water) and maintaining the temperature thereof at 39°C.

The amount of the sodium neutralizer added up to the elapse of 10.3 hours from the start of the reaction was 219 g. The concentration of succinic acid measured at this time was 72.8 g/L, and the concentration of disodium succinate was 617 mM.

At this point in time, the sodium neutralizer was changed to an ammonia neutralizer (106.5 g of ammonium hydrogen carbonate, 221.5 g of 13.8 N ammonia water, and 704.1 g of sterilized water).

The concentration of succinic acid measured at the time of the elapse of 12.0 hours from the start of the reaction was 79.0 g/L. Table 9 shows a succinic acid production rate represented by an average value during a reaction time from the changing of the neutralizer to the measurement of the succinic acid concentration.

### [Comparative Example 7]

Succinic acid production reaction was performed in the same manner as Example 8 except that the sodium neutralizer first used for the pH adjustment of the aqueous medium in (C) the succinic acid production reaction was changed to an ammonia neutralizer (106.5 g of ammonium hydrogen carbonate, 221.5 g of 13.8 N ammonia water, and 704.1 g of sterilized water) and there was no changing of the neutralizer after that.

Succinic acid concentrations measured at the times of the elapses of 10.3 hours, 12.0 hours, 18.7 hours, and 24.0 hours (corresponding to Example 8), respectively, from the start of the reaction were 57.7 g/L, 63.2 g/L, 70.8 g/L, and 77.0 g/L, respectively.

Table 9 shows a succinic acid production rate as the result of Comparative Example 7, which is corresponding to Example 8.

In Table 9, the succinic acid production rate (g/L/h) was calculated from succinic acid concentrations (g/L) at respective two points in time shown in the Table. The succinic acid production rate is represented by an average value during a reaction time from 18.7 hours to 24.0 hours, in which succinic acid concentrations are approximately the same as those measured at the calculation of the succinic acid production rate in Example 8.

### [Comparative Example 8]

Succinic acid production reaction was performed in the same manner as Example 8 except for not changing the neutralizer. Succinic acid concentrations measured at the times of the elapses of 10.3 hours and 12.0 hours (corresponding to Example 8), respectively, from the start of the reaction were 71.7 g/L and 75.8 g/L, respectively.

Table 9 shows a succinic acid production rate as the result of Comparative Example 8, which is corresponding to Example 8.

**Table 9**

| | Kind of neutralizer | Disodium succinate concentration after 10.3 hours [mM] | Succinic acid concentration after 10.3 hours [g/L] | Succinic acid concentration after 12.0 hours [g/L] | Reaction time [h] | Succinic acid production rate [g/L/h] |
|---|---|---|---|---|---|---|
| Example 8 | Sodium neutralizer ⇒ Ammonia neutralizer | 617 | 72.8 | 79.0 | 1.7 | 3.65 |
| Comparative Example 7 | Ammonia neutralizer | 0 (after 18.7 hours) | 70.8 (after 18.7 hours) | 77.0 (after 24.0 hours) | 5.3 | 1.17 |
| Comparative Example 8 | Sodium neutralizer | 607 | 71.7 | 75.8 | 1.7 | 2.41 |

Table 9 shows that Example 8 improved the succinic acid production rate by 212% as compared to Comparative Example 7 and by 51 % as compared to Comparative Example 8.

The above results demonstrated that the condition of using the sodium neutralizer until the accumulation of disodium succinate of 617 mM and then changing to the ammonia neutralizer improved the succinic acid production rate as compared to the condition of using the ammonia neutralizer alone and the condition of using the sodium neutralizer alone.

## Claims

1. A method for producing succinic acid comprising:
(A) a step of converting an organic raw material to succinic acid in the presence of a microorganism or a processed product thereof in an aqueous medium and
(B) a step of recovering the succinic acid, wherein (A) the step of converting an organic raw material to succinic acid comprises:
(a1) a step of adjusting the concentration of a succinic acid alkali metal salt in the aqueous medium to 80 mM or more; and then,
(a2) a step of adding ammonia and/or an ammonium salt in the aqueous medium.

2. The method for producing succinic acid according to claim 1, wherein (a1) the step of adjusting the concentration of a succinic acid alkali metal salt in the aqueous medium to 80 mM or more is a step of neutralizing the aqueous medium with an alkali metal salt and/or an alkali metal hydroxide.

3. The method for producing succinic acid according to claim 1 or 2, wherein in (a1) the step of adjusting the concentration of a succinic acid alkali metal salt in the aqueous medium to 80 mM or more, the concentration of the succinic acid alkali metal salt is 1300 mM or less.

4. The method for producing succinic acid according to any one of claims 1 to 3, wherein the succinic acid alkali metal salt is disodium succinate and/or dipotassium succinate.

5. The method for producing succinic acid according to any one of claims 1 to 4, wherein the microorganism is at least one selected from the group consisting of coryneform bacteria, *Escherichia coli,* the genus *Anaerobiospirillum*, the genus *Actinobacillus*, filamentous fungi, and yeast.

6. The method for producing succinic acid according to any one of claims 1 to 5, wherein the microorganism is a microorganism modified such that lactate dehydrogenase activity is reduced compared to an unmodified strain.

7. The method for producing succinic acid according to any one of claims 1 to 6, wherein the microorganism is a microorganism modified such that pyruvate carboxylase activity is enhanced compared to an unmodified strain.

8. The method for producing succinic acid according to any one of claims 1 to 7, wherein the aqueous medium contains at least one selected from the group consisting of a carbonate ion, a bicarbonate ion, and carbon dioxide gas.

9. The method for producing succinic acid according to any one of claims 1 to 8, wherein (A) the step of converting an organic raw material to succinic acid is performed in an anaerobic atmosphere.

10. The method for producing succinic acid according to any one of claims 1 to 9, wherein (A) the step of converting an organic raw material to succinic acid is performed by batch reaction or semi-batch reaction.

11. The method for producing succinic acid according to any one of claims 1 to 10, wherein the organic raw material is glucose and/or sucrose.

12. A method for producing a succinic acid-containing polymer comprising a step of producing succinic acid by the method according to any one of claims 1 to 11 and a step of polymerizing the obtained succinic acid.


SEQUENCE LISTING

<110>  Mitsubishi Chemical Corporation

<120>  Method for producing succinic acid

<130>  X1857 EP S3

<140>  EP 12 84 7315.4
<141>  2012-11-09

<150>  PCT/JP2012/079172
<151>  2012-11-09

<150>  JP2011-248101
<151>  2011-11-11

<160>  14

<170>  PatentIn version 3.5

<210>  1
<211>  34
<212>  DNA
<213>  Artificial Sequence

<220>
<223>  primer

<400>  1
aaacgacaat aaagtaaggc tatcctaata agtg                                34


<210>  2
<211>  24
<212>  DNA
<213>  Artificial Sequence

<220>
<223>  primer

<400>  2
gtgacaggtg gacgaacagg acca                                           24


<210>  3
<211>  27
<212>  DNA
<213>  Artificial Sequence

<220>
<223>  primer

<400>  3
gacactggag gtgccatgac tgttaga                                        27


<210>  4
<211>  24
<212>  DNA
<213>  Artificial Sequence

<220>

<223>  primer

<400>  4
acacaggaaa cagctatgac catg                                              24


<210>  5
<211>  36
<212>  DNA
<213>  Artificial Sequence

<220>
<223>  primer

<400>  5
gtggatgaga caggactatc tagagctaca gtgaca                                 36


<210>  6
<211>  33
<212>  DNA
<213>  Artificial Sequence

<220>
<223>  primer

<400>  6
agaattgatc ttaggtcact aaaactaatt cag                                    33


<210>  7
<211>  22
<212>  DNA
<213>  Artificial Sequence

<220>
<223>  primer

<400>  7
gcgtacgtcg tgctcaatct ga                                                22


<210>  8
<211>  25
<212>  DNA
<213>  Artificial Sequence

<220>
<223>  primer

<400>  8
actgtcgttg ttactgtacg tccca                                             25


<210>  9
<211>  24
<212>  DNA
<213>  Artificial Sequence

<220>
<223>  primer

<400>  9

actggcattg atgtcgatcc agca                                              24

<210>  10
<211>  23
<212>  DNA
<213>  Artificial Sequence

<220>
<223>  primer

<400>  10
atgaattccc cgcagaacgt ttc                                               23

<210>  11
<211>  20
<212>  DNA
<213>  Artificial Sequence

<220>
<223>  primer

<400>  11
gttaaagatt agagcaagtc                                                   20

<210>  12
<211>  27
<212>  DNA
<213>  Artificial Sequence

<220>
<223>  primer

<400>  12
agttttgggt gatctagacg atactgc                                           27

<210>  13
<211>  27
<212>  DNA
<213>  Artificial Sequence

<220>
<223>  primer

<400>  13
aggtatataa agcgtcgacg attttgc                                           27

<210>  14
<211>  26
<212>  DNA
<213>  Artificial Sequence

<220>
<223>  primer

<400>  14
gaactgccga gctccgagcc cgtgaa                                            26